# EUROPEAN PATENT SPECIFICATION

(11) **EP 0 979 310 B1**
(45) Date of publication and mention of the grant of the patent: **13.03.2002**
(21) Application number: 98920316.1
(22) Date of filing: 06.05.1998
(51) Int. Cl.: C12Q 1/68

(54) **MUTATIONS IN THE katG GENE USEFUL FOR DETECTION OF M. TUBERCULOSIS**
MUTATIONEN IM katG GEN DIE NÜTZLICH SIND ZUM NACHWEIS VON M. TUBERCULOSIS
MUTATIONS DU GENE katG UTILES A LA DETECTION DE M. TUBERCULOSIS

(30) Priority: 07.05.1997 US 852219
(43) Date of publication of application: 16.02.2000
(73) Proprietor: MAYO FOUNDATION FOR MEDICAL EDUCATION AND RESEARCH, Rochester, MN 55905 (US)
(72) Inventor: COCKERILL, Franklin, R., III, Rochester, MN 55902 (US); KLINE, Bruce, C., Rochester, MN 55902 (US); UHL, James, R., Rochester, MN 55902 (US)
(74) Representative: VOSSIUS & PARTNER
(86) International application number: PCT/US98/09285
(87) International publication number: WO 98/50585

(56) References cited:
- WO-A-93/22454
- WO-A-96/15267
- TEMESGEN Z ET AL: "Use of polymerase chain reaction single-strand conformation polymorphism (PCR-SSCP) analysis to detect a point mutation in the catalase-peroxidase gene (KATG) of mycobacterium tuberculosis" MOLECULAR AND CELLULAR PROBES, vol. 11, no. 1, February 1997, page 59-63 XP002075616
- MARTTILA H ET AL: "KATG mutations in isoniazid-resistant mycobacterium tuberculosis isolates recovered from finnish patients" ANTIMICROBIAL AGENTS AND CHEMOTHERAPY, vol. 40, no. 9, - September 1996 pages 2187-9, XP002075617
- COCKERILL F ET AL: "Rapid identification of a point mutation of the mycobacterium tuberculosis catalase-peroxidase (KATG) gene associated with isoniazid resistance" JOURNAL OF INFECTIOUS DISEASES , vol. 171 , no. 1, January 1995, pages 240-5, XP002075618

### SEQUENCE LISTING

### (1) GENERAL INFORMATION:

(i) APPLICANT: Cockerill, Franklin R.
   Kline, Bruce C.
   Uhl, James R.
(ii) TITLE OF INVENTION: Detection of Isoniazid Resistant Strains of M. Tuberculosis
(iii) NUMBER OF SEQUENCES: 22
(iv) CORRESPONDENCE ADDRESS:
   (A) ADDRESSEE: Schwegman, Lundberg & Woessner
   (B) STREET: 3500 IDS Center
   (C) CITY: Minneapolis
   (D) STATE: MN
   (E) COUNTRY: USA
   (F) ZIP: 55402
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25
(vi) CURRENT APPLICATION DATA:
   (A) APPLICATION NUMBER: US
   (B) FILING DATE:
   (C) CLASSIFICATION:
(viii) ATTORNEY/AGENT INFORMATION:
   (A) NAME: Woessner, Warren D.
   (B) REGISTRATION NUMBER: 30,440
   (C) REFERENCE/DOCKET NUMBER: 150.185US1
(ix) TELECOMMUNICATION INFORMATION:
   (A) TELEPHONE: 612-339-0331
   (B) TELEFAX: 612-339-3061

### (2) INFORMATION FOR SEQ ID NO:1:

(i) SEQUENCE CHARACTERISTICS:
   (A) LENGTH: 2235 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:1:
   AGGAATGCTG TGCCCGAGCA ACACCCACCC ATTACAGAAA CCACCACCGG AGCCGCTAGC 60
   AACGGCTGTC CCGTCGTGGG TCATATGAAA TACCCCGTCG AGGGCGGCGG AAACCAGGAC 120
   TGGTGGCCCA ACCGGCTCAA TCTGAAGGTA CTGCACCAAA ACCCGGCCGT CGCTGACCCG 180
   ATGGGTGCGG CGTTCGACTA TGCCGCGGAG GTCGCGACCA TCGACGTTGA CGCCCTGACG 240
   CGGGACATCG AGGAAGTGAT GACCACCTCG CAGCCGTGGT GGCCCGCCGA CTACGGCCAC 300
   TACGGGCCGC TGTTTATCCG GATGGCGTGG CACGCTGCCG GCACCTACCG CATCCACGAC 360
   GGCCGCGGCG GCGCCGGGGG CGGCATGCAG CGGTTCGCGC CGCTTAACAG CTGGCCCGAC 420
   AACGCCAGCT TGGACAAGGC GCGCCGGCTG CTGTGGCCGG TCAAGAAGAA GTACGGCAAG 480
   AAGCTCTCAT GGGCGGACCT GATTGTTTTC GCCGGCAACT GCGCGCTGGA ATCGATGGGC 540
   TTCAAGACGT TCGGGTTCGG CTTCGGCCGG GTCGACCAGT GGGAGCCCGA TGAGGTCTAT 600
   TGGGGCAAGG AAGCCACCTG GCTCGGCGAT GAGCGTTACA GCGGTAAGCG GGATCTGGAG 660
   AACCCGCTGG CCGCGGTGCA GATGGGGCTG ATCTACGTGA ACCCGGAGGG GCCGAACGGC 720
   AACCCGGACC CCATGGCCGC GGCGGTCGAC ATTCGCGAGA CGTTTCGGCG CATGGCCATG 780
   AACGACGTCG AAACAGCGGC GCTGATCGTC GGCGGTCACA CTTTCGGTAA GACCCATGGC 840
   GCCGGCCCGG CCGATCTGGT CGGCCCCGAA CCCGAGGCTG CTCCGCTGGA GCAGATGGGC
   900
   TTGGGCTGGA AGAGCTCGTA TGGCACCGGA ACCGGTAAGG ACGCGATCAC CAGCGGCATC
   960
   GAGGTCGTAT GGACGAACAC CCCGACGAAA TGGGACAACA GTTTCCTCGA GATCCTGTAC
   1020
   GGCTACGAGT GGGAGCTGAC GAAGAGCCCT GCTGGCGCTT GGCAATACAC CGCCAAGGAC
   1080
   GGCGCCGGTG CCGGCACCAT CCCGGACCCG TTCGGCGGGC CAGGGCGCTC CCCGACGATG
   1140
   CTGGCCACTG ACCTCTCGCT GCGGGTGGAT CCGATCTATG AGCGGATCAC GCGTCGCTGG
   1200
   CTGGAACACC CCGAGGAATT GGCCGACGAG TTCGCCAAGG CCTGGTACAA GCTGATCCAC
   1260
   CGAGACATGG GTCCCGTTGC GAGATACCTT GGGCCGCTGG TCCCCAAGCA GACCCTGCTG
   1320
   TGGCAGGATC CGGTCCCTGC GGTCAGCCAC GACCTCGTCG GCGAAGCCGA GATTGCCAGC
   1380
   CTTAAGAGCC AGATCCGGGC ATCGGGATTG ACTGTCTCAC AGCTAGTTTC GACCGCATGG
   1440
   GCGGCGGCGT CGTCGTTCCG TGGTAGCGAC AAGCGCGGCG GCGCCAACGG TGGTCGCATC
   1500
   CGCCTGCAGC CACAAGTCGG GTGGGAGGTC AACGACCCCG ACGGGGATCT GCGCAAGGTC
   1560
   ATTCGCACCC TGGAAGAGAT CCAGGAGTCA TTCAACTCCG CGGCGCCGGG GAACATCAAA
   1620
   GTGTCCTTCG CCGACCTCGT CGTGCTCGGT GGCTGTGCCG CCATAGAGAA AGCAGCAAAG
   1680
   GCGGCTGGCC ACAACATCAC GGTGCCCTTC ACCCCGGGCC GCACGGATGC GTCGCAGGAA
   1740
   CAAACCGACG TGGAATCCTT TGCCGTGCTG GAGCCCAAGG CAGATGGCTT CCGAAACTAC
   1800
   CTCGGAAAGG GCAACCCGTT GCCGGCCGAG TACATGCTGC TCGACAAGGC GAACCTGCTT
   1860
   ACGCTCAGTG CCCCTGAGAT GACGGTGCTG GTAGGTGGCC TGCGCGTCCT CGGGCAAACT
   1920
   ACAAGCGCTT ACCGCTGGGC GTGTTCACCG AGGCCTCCGA GTCACTGACC AACGACTTCT
   1980
   TCGTGAACCT GCTCGACATG GGTATCACCT GGGAGCCCTC GCCAGCAGAT GACGGGACCT
   2040
   ACCAGGGCAA GGATGGCAGT GGCAAGGTGA AGTGGACCGG CAGCCGCGTG GACCTGGTCT
   2100
   TCGGGTCCAA CTCGGAGTTG CGGGCGCTTG TCGAGGTCTA TGGCGCCGAT GACGCGCAGC
   2160
   CGAAGTTCGT GCAGGACTTC GTCGCTGCCT GGGACAAGGT GATGAACCTC GACAGGTTCG
   2220
   ACGTGCGCTG ATTCG
   2235

### (2) INFORMATION FOR SEQ ID NO:2:

(i) SEQUENCE CHARACTERISTICS:
   (A) LENGTH: 2221 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:2:
   AGGAATGCTG TGCCCGAGCA ACACCCACCC ATTACAGAAA CCACCACCGG AGCCGCTAGC
   60
   AACGGCTGTC CCGTCGTGGG TCATATGAAA TACCCCGTCG AGGGCGGCGG AAACCAGGAC
   120
   TGGTGGCCCA ACCGGCTCAA TCTGAAGGTA CTGCACCAAA ACCCGGCCGT CGCTGACCCG
   180
   ATGGGTGCGG CGTTCGACTA TGCCGCGGAG GTCGCGACCA GTCGACTTGA CGCCCTGACG
   240
   CGGGACATCG AGGAAGTGAT GACCACCTCG CAGCCGTGGT GGCCCGCCGA CTACGGCCAC
   300
   TACGGGCCGC TGTTTATCCG GATGGCGTGG CACGCTGCCG GCACCTACCG CATCCACGAC
   360
   GGCCGCGGCG GCGCCGGGGG CGGCATGCAG CGGTTCGCGC CGCTTAACAG CTGGCCCGAC
   420
   AACGCCAGCT TGGACAAGGC GCGCCGGCTG CTGTGGCCGG TCAAGAAGAA GTACGGCAAG
   480
   AAGCTCTCAT GGGCGGACCT GATTGTTTTC GCCGGCAACC GCTGCGCTCG GAATCGATGG
   540
   GCTTCAAGAC GTTCGGGTTC GGCTTCGGGC GTCGACCAGT GGGAGACCGA TGAGGTCTAT
   600
   TGGGGCAAGG AAGCCACCTG GCTCGGCGAT GACGGTTACA GCGTAAGCGA TCTGGAGAAC
   660
   CCGCTGGCCG CGGTGCAGAT GGGGCTGATC TACGTGAACC CGGAGGCGCC GAACGGCAAC
   720
   CCGGACCCCA TGGCCGCGGC GGTCGACATT CGCGAGACGT TTCGGCGCAT GGCCATGAAC
   780
   GACGTCGAAA CAGCGGCGCT GATCGTCGGC GGTCACACTT TCGGTAAGAC CCATGGCGCC
   840
   GGCCCGGCCG ATCTGGTCGG CCCCGAACCC GAGGCTGCTC CGCTGGAGCA GATGGGCTTG
   900
   GGCTGGAAGA GCTCGTATGG CACCGGAACC GGTAAGGACG CGATCACCAG CGGCATCGAG
   960
   GTCGTATGGA CGAACACCCC GACGAAATGG GACAACAGTT TCCTCGAGAT CCTGTACGGC
   1020
   TACGAGTGGG AGCTGACGAA GAGCCCTGCT GGCGCTTGGC AATACACCGC CAAGGACGGC
   1080
   GCCGGTGCCG GCACCATCCC GGACCCGTTC GGCGGGCCAG GGCGCTCCCC GACGATGCTG
   1140
   GCCACTGACC TCTCGCTGCG GGTGGATCCG ATCTATGAGC GGATCACGCG TCGCTGGCTG
   1200
   GAACACCCCG AGGAATTGGC CGACGAGTTC CGCAAGGCCT GGTACAAGCT GATCCACCGA
   1260
   GACATGGGTC CCGTTGCGAG ATACCTTGGG CCGCTGGTCC CCAAGCAGAC CCTGCTGTGG
   1320
   CAGGATCCGG TCCCTGCGGT CAGCACGACC TCGTCGGCGA AGCAGATTGC CAGCCTTAAG
   1380
   AGCCAGATCC GGGCATCGGG ATTGACTGTC TCACAGCTAG TTTCGACCGC ATGGGCGGCG
   1440
   GCGTCGTCGT TCCGTGGTAG CGACAAGCGC GGCGGCGCCA ACGGTGGTCG CATCCGCCTG
   1500
   CAGCCACAAG TCGGGTGGGA GGTCAACGAC CCCGACGGAT CTGCGCAAGG TCATTCGCAC
   1560
   CCTGAAGAGA TCCAGGAGTC ATTCACTCGG CGCGGGAACA TCAAAGTGTC CTTCGCCGAC
   1620
   CTCGTCGTGC TCGGTGGCTG TGCGCCACTA GAGAAAGCAG CAAAGGCGGC TGGCCACAAC
   1680
   ATCACGGTGC CCTTCACCCC GGGCCCGCAC GATGCGTCGC AGGAACAAAC CGACGTGGAA
   1740
   TCCTTTGCCG TGCTGGAGCC CAAGGCAGAT GGCTTCCGAA ACTACCTCGG AAAGGGCAAC
   1800
   CGTTGCCGGC CGAGTACATC GCTGCTCGAC AAGGCGAACC TGCTTACGCT CAGTGCCCCT
   1860
   GAGATGACGG TGCTGGTAGG TGGCCTGCGC GTCCTCGGCG CAAACTACAA GCGCTTACCG
   1920
   CTGGGCGTGT TCACCGAGGC CTCCGAGTCA CTGACCAACG ACTTCTTCGT GAACCTGCTC
   1980
   GACATGGGTA TCACCTGGGA GCCCTCGCCA GCAGATGACG GGACCTACCA GGGCAAGGAT
   2040
   GGCAGTGGCA AGGTGAAGTG GACCGGCAGC CGCGTGGACC TGGTCTTCGG GTCCAACTCG
   2100
   GAGTTGCGGG CGCTTGTCGA GGTCTATGCG CCGATGACGC GGCAGGCGAA GTTCGTGACA
   2160
   GGATTCGTCG CTGCGTGGGA CAAGGTGATG AACCTCGACA GGTTCGACGT GCGCTGATTC
   2220
   G
   2221

### (2) INFORMATION FOR SEQ ID NO:3:

(i) SEQUENCE CHARACTERISTICS:
   (A) LENGTH: 30 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:3:
   TCGGACCATA ACGGCTTCCT GTTGGACGAG
   30

### (2) INFORMATION FOR SEQ ID NO:4:

(i) SEQUENCE CHARACTERISTICS:
   (A) LENGTH: 30 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:4:
   AATCTGCTTC GCCGACGAGG TCGTGCTGAC
   30

### (2) INFORMATION FOR SEQ ID NO:5:

(i) SEQUENCE CHARACTERISTICS:
   (A) LENGTH: 30 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:5:
   CACCCCGACG AAATGGGACA ACAGTTTCCT
   30

### (2) INFORMATION FOR SEQ ID NO:6:

(i) SEQUENCE CHARACTERISTICS:
   (A) LENGTH: 30 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:6:
   GGGTCTGACA AATCGCGCCG GGCAAACACC
   30

### (2) INFORMATION FOR SEQ ID NO:7:

(i) SEQUENCE CHARACTERISTICS:
   (A) LENGTH: 740 amino acids
   (B) TYPE: amino acid
   (C) STRANDEDNESS: single
   (D) TOPOLOGY: linear
(ii) MOLECULE TYPE: protein
(xi) SEQUENCE DESCRIPTION: SEQ ID NO:7:
   Val Pro Glu Gly His Pro Pro Ile Thr Glu Thr Thr Thr Gly Ala Ala
   1               5                   10                  15
   Ser Asn Gly Cys Pro Val Val Gly His Met Lys Tyr Pro Val Glu Gly
               20                  25                  30
   Gly Gly Asn Gln Asp Trp Trp Pro Asn Arg Leu Asn Leu Lys Val Leu
           35                  40                  45
   His Gln Asn Pro Ala Val Ala Asp Pro Met Gly Ala Ala Phe Asp Tyr
       50                  55                  60
   Ala Ala Glu Val Ala Thr Ile Asp Val Asp Ala Leu Thr Arg Asp Ile
   65                  70                  75                  80
   Glu Glu Val Met Thr Thr Ser Gln Pro Trp Trp Pro Ala Asp Tyr Gly
                   85                  90                  95
   His Tyr Gly Pro Leu Phe Ile Arg Met Ala Trp His Ala Ala Gly Thr
               100                 105                 110
   Tyr Arg Ile His Asp Gly Arg Gly Gly Ala Gly Gly Gly Met Gln Arg
           115                 120                 125
   Phe Ala Pro Leu Asn Ser Trp Pro Asp Asn Ala Ser Leu Asp Lys Ala
       130                 135                 140
   Arg Arg Leu Leu Trp Pro Val Lys Lys Lys Tyr Gly Lys Lys Leu Ser
   145                 150                 155                 160
   Trp Ala Asp Leu Ile Val Phe Ala Gly Asn Cys Ala Leu Glu Ser Met
                   165                 170                 175
   Gly Phe Lys Thr Phe Gly Phe Gly Phe Gly Arg Val Asp Gln Trp Glu
               180                 185                 190
   Pro Asp Glu Val Tyr Trp Gly Lys Glu Ala Thr Trp Leu Gly Asp Glu
           195                 200                 205
   Arg Tyr Ser Gly Lys Arg Asp Leu Glu Asn Pro Leu Ala Ala Val Gln
       210                 215                 220
   Met Gly Leu Ile Tyr Val Asn Pro Glu Gly Pro Asn Gly Asn Pro Asp
   225                 230                 235                 240
   Pro Met Ala Ala Ala Val Asp Ile Arg Glu Thr Phe Arg Arg Met Ala
                   245                 250                 255
   Met Asn Asp Val Glu Thr Ala Ala Leu Ile Val Gly Gly His Thr Phe
               260                 265                 270
   Gly Lys Thr His Gly Ala Gly Pro Ala Asp Leu Val Gly Pro Glu Pro
           275                 280                 285
   Glu Ala Ala Pro Leu Glu Gln Met Gly Leu Gly Trp Lys Ser Ser Tyr
       290                 295                 300
   Gly Thr Gly Thr Gly Lys Asp Ala Ile Thr Ser Gly Ile Glu Val Val
   305                 310                 315                 320
   Trp Thr Asn Thr Pro Thr Lys Trp Asp Asn Ser Phe Leu Glu Ile Leu
                   325                 330                 335
   Tyr Gly Tyr Glu Trp Glu Leu Thr Lys Ser Pro Ala Gly Ala Trp Gln
               340                 345                 350
   Tyr Thr Ala Lys Asp Gly Ala Gly Ala Gly Thr Ile Pro Asp Pro Phe
           355                 360                 365
   Gly Gly Pro Gly Arg Ser Pro Thr Met Leu Ala Thr Asp Leu Ser Leu
       370                 375                 380
   Arg Val Asp Pro Ile Tyr Glu Arg Ile Thr Arg Arg Trp Leu Glu His
   385                 390                 395                 400
   Pro Glu Glu Leu Ala Asp Glu Phe Ala Lys Ala Trp Tyr Lys Leu Ile
                   405                 410                 415
   His Arg Asp Met Gly Pro Val Ala Arg Tyr Leu Gly Pro Leu Val Pro
   420 425 430
   Lys Gln Thr Leu Leu Trp Gln Asp Pro Val Pro Ala Val Ser His Asp
   435 440 445
   Leu Val Gly Glu Ala Glu Ile Ala Ser Leu Lys Ser Gln Ile Arg Ala
   450 455 460
   Ser Gly Leu Thr Val Ser Gln Leu Val Ser Thr Ala Trp Ala Ala Ala
   465 470 475 480
   Ser Ser Phe Arg Gly Ser Asp Lys Arg Gly Gly Ala Asn Gly Gly Arg
   485 490 495
   Ile Arg Leu Gln Pro Gln Val Gly Trp Glu Val Asn Asp Pro Asp Gly
   500 505 510
   Asp Leu Arg Lys Val Ile Arg Thr Leu Glu Glu Ile Gln Glu Ser Phe
   515 520 525
   Asn Ser Ala Ala Pro Gly Asn Ile Lys Val Ser Phe Ala Asp Leu Val
   530 535 540
   Val Leu Gly Gly Cys Ala Ala Ile Glu Lys Ala Ala Lys Ala Ala Gly
   545 550 555 560
   His Asn Ile Thr Val Pro Phe Thr Pro Gly Arg Thr Asp Ala Ser Gln
   565 570 575
   Glu Gln Thr Asp Val Glu Ser Phe Ala Val Leu Glu Pro Lys Ala Asp
   580 585 590
   Gly Phe Arg Asn Tyr Leu Gly Lys Gly Asn Pro Leu Pro Ala Glu Tyr
   595 600 605
   Met Leu Leu Asp Lys Ala Asn Leu Leu Thr Leu Ser Ala Pro Glu Met
   610 615 620
   Thr Val Leu Val Gly Gly Leu Arg Val Leu Gly Ala Asn Tyr Lys Arg
   625 630 635 640
   Leu Pro Leu Gly Val Phe Thr Glu Ala Ser Glu Ser Leu Thr Asn Asp
   645 650 655
   Phe Phe Val Asn Leu Leu Asp Met Gly Ile Thr Trp Glu Pro Ser Pro
   660 665 670
   Ala Asp Asp Gly Thr Tyr Gln Gly Lys Asp Gly Ser Gly Lys Val Lys
   675 680 685
   Trp Thr Gly Ser Arg Val Asp Leu Val Phe Gly Ser Asn Ser Glu Leu
   690 695 700
   Arg Ala Leu Val Glu Val Tyr Gly Ala Asp Asp Ala Gln Pro Lys Phe
   705 710 715 720
   Val Gln Asp Phe Val Ala Ala Trp Asp Lys Val Met Asn Leu Asp Arg
   725 730 735
   Phe Asp Val Arg
   740

### (2) INFORMATION FOR SEQ ID NO:8:

(i) SEQUENCE CHARACTERISTICS:
   (A) LENGTH: 27 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:8:
   GTCGAAACAG CGGCGCTGAT CGTCGGC
   27

### (2) INFORMATION FOR SEQ ID NO:9:

(i) SEQUENCE CHARACTERISTICS:
   (A) LENGTH: 27 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:9:
   GTCGAAACAG CGACGCTGAT CGTCGGC
   27

### (2) INFORMATION FOR SEQ ID NO:10:

(i) SEQUENCE CHARACTERISTICS:
   (A) LENGTH: 27 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:10:
   CTCGAGATCC TGTACGGCTA CGAGTGG
   27

### (2) INFORMATION FOR SEQ ID NO:11:

(i) SEQUENCE CHARACTERISTICS:
   (A) LENGTH: 27 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:11:
   CTCGAGATCC TGTGCGGCTA CGAGTGG
   27

### (2) INFORMATION FOR SEQ ID NO:12:

(i) SEQUENCE CHARACTERISTICS:
   (A) LENGTH: 27 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:12:
   GACGCGATCA CCAGCGGCAT CGAGGTC
   27

### (2) INFORMATION FOR SEQ ID NO:13:

(i) SEQUENCE CHARACTERISTICS:
   (A) LENGTH: 27 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:13:
   GACGCGATCA CCACCGGCAT CGAGGTC
   27

### (2) INFORMATION FOR SEQ ID NO:14:

(i) SEQUENCE CHARACTERISTICS:
   (A) LENGTH: 27 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:14:
   AAGAGCCAGA TCCGGGCATC GGGATTG
   27

### (2) INFORMATION FOR SEQ ID NO:15:

(i) SEQUENCE CHARACTERISTICS:
   (A) LENGTH: 27 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:15:
   AAGAGCCAGA TCCTGGCATC GGGATTG
   27

### (2) INFORMATION FOR SEQ ID NO:16:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:16:
   AGCTCGTATG GCACCGGAAC
   20

### (2) INFORMATION FOR SEQ ID NO:17:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:17:
   TTGACCTCCC ACCCGACTTG
   20

### (2) INFORMATION FOR SEQ ID NO:18:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:18:
   CGGTAAGCGG GATCTGGAGA
   20

### (2) INFORMATION FOR SEQ ID NO:19:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:19:
   CATTTCGTCG GGGTGTTCGT
   20

### (2) INFORMATION FOR SEQ ID NO:20:

(i) SEQUENCE CHARACTERISTICS:
   (A) LENGTH: 2331 base pairs
   (B) TYPE: nucleic acid
   (C) STRANDEDNESS: single
   (D) TOPOLOGY: linear
(ii) MOLECULE TYPE: DNA
(ix) FEATURE:
   (A) NAME/KEY: CDS
   (B) LOCATION: 70..2289
(xi) SEQUENCE DESCRIPTION: SEQ ID NO:20:
   CGATATCCGA CACTTCGCGA TCACATCCGT GATCACAGCC CGATAACACC AACTCCTGGA
   60
   AGGAATGCT GTG CCC GAG CAA CAC CCA CCC ATT ACA GAA ACC ACC ACC
   108
             Val Pro Glu Gln His Pro Pro Ile Thr Glu Thr Thr Thr
               1               5                  10
   GGA GCC GCT AGC AAC GGC TGT CCC GTC GTG GGT CAT ATG AAA TAC CCC
   156
   Gly Ala Ala Ser Asn Gly Cys Pro Val Val Gly His Met Lys Tyr Pro
        15                  20                  25
   GTC GAG GGC GGC GGA AAC CAG GAC TGG TGG CCC AAC CGG CTC AAT CTG
   204
   Val Glu Gly Gly Gly Asn Gln Asp Trp Trp Pro Asn Arg Leu Asn Leu
    30                  35                  40                  45
   AAG GTA CTG CAC CAA AAC CCG GCC GTC GCT GAC CCG ATG GGT GCG GCG
   252
   Lys Val Leu His Gln Asn Pro Ala Val Ala Asp Pro Met Gly Ala Ala
                    50                  55                  60
   TTC GAC TAT GCC GCG GAG GTC GCG ACC ATC GAC GTT GAC GCC CTG ACG
   300
   Phe Asp Tyr Ala Ala Glu Val Ala Thr Ile Asp Val Asp Ala Leu Thr
                65                  70                  75
   CGG GAC ATC GAG GAA GTG ATG ACC ACC TCG CAG CCG TGG TGG CCC GCC
   348
   Arg Asp Ile Glu Glu Val Met Thr Thr Ser Gln Pro Trp Trp Pro Ala
            80                  85                  90
   GAC TAC GGC CAC TAC GGG CCG CTG TTT ATC CGG ATG GCG TGG CAC GCT
   396
   Asp Tyr Gly His Tyr Gly Pro Leu Phe Ile Arg Met Ala Trp His Ala
        95                 100                 105
   GCC GGC ACC TAC CGC ATC CAC GAC GGC CGC GGC GGC GCC GGG GGC GGC
   444
   Ala Gly Thr Tyr Arg Ile His Asp Gly Arg Gly Gly Ala Gly Gly Gly
   110                 115                 120                 125
   ATG CAG CGG TTC GCG CCG CTT AAC AGC TGG CCC GAC AAC GCC AGC TTG
   492
   Met Gln Arg Phe Ala Pro Leu Asn Ser Trp Pro Asp Asn Ala Ser Leu
                   130                 135                 140
   GAC AAG GCG CGC CGG CTG CTG TGG CCG GTC AAG AAG AAG TAC GGC AAG
   540
   Asp Lys Ala Arg Arg Leu Leu Trp Pro Val Lys Lys Lys Tyr Gly Lys
               145                 150                 155
   AAG CTC TCA TGG GCG GAC CTG ATT GTT TTC GCC GGC AAC TGC GCG CTG 588
   Lys Leu Ser Trp Ala Asp Leu Ile Val Phe Ala Gly Asn Cys Ala Leu
   160 165 170
   GAA TCG ATG GGC TTC AAG ACG TTC GGG TTC GGC TTC GGC CGG GTC GAC 636
   Glu Ser Met Gly Phe Lys Thr Phe Gly Phe Gly Phe Gly Arg Val Asp
   175 180 185
   CAG TGG GAG CCC GAT GAG GTC TAT TGG GGC AAG GAA GCC ACC TGG CTC 684
   Gln Trp Glu Pro Asp Glu Val Tyr Trp Gly Lys Glu Ala Thr Trp Leu
   190 195 200 205
   GGC GAT GAG CGT TAC AGC GGT AAG CGG GAT CTG GAG AAC CCG CTG GCC 732
   Gly Asp Glu Arg Tyr Ser Gly Lys Arg Asp Leu Glu Asn Pro Leu Ala
   210 215 220
   GCG GTG CAG ATG GGG CTG ATC TAC GTG AAC CCG GAG GGG CCG AAC GGC 780
   Ala Val Gln Met Gly Leu Ile Tyr Val Asn Pro Glu Gly Pro Asn Gly
   225 230 235
   AAC CCG GAC CCC ATG GCC GCG GCG GTC GAC ATT CGC GAG ACG TTT CGG 828
   Asn Pro Asp Pro Met Ala Ala Ala Val Asp Ile Arg Glu Thr Phe Arg
   240 245 250
   CGC ATG GCC ATG AAC GAC GTC GAA ACA GCG GCG CTG ATC GTC GGC GGT 876
   Arg Met Ala Met Asn Asp Val Glu Thr Ala Ala Leu Ile Val Gly Gly
   255 260 265
   CAC ACT TTC GGT AAG ACC CAT GGC GCC GGC CCG GCC GAT CTG GTC GGC 924
   His Thr Phe Gly Lys Thr His Gly Ala Gly Pro Ala Asp Leu Val Gly
   270 275 280 285
   CCC GAA CCC GAG GCT GCT CCG CTG GAG CAG ATG GGC TTG GGC TGG AAG 972
   Pro Glu Pro Glu Ala Ala Pro Leu Glu Gln Met Gly Leu Gly Trp Lys
   290 295 300
   AGC TCG TAT GGC ACC GGA ACC GGT AAG GAC GCG ATC ACC AGC GGC ATC 1020
   Ser Ser Tyr Gly Thr Gly Thr Gly Lys Asp Ala Ile Thr Ser Gly Ile
   305 310 315
   GAG GTC GTA TGG ACG AAC ACC CCG ACG AAA TGG GAC AAC AGT TTC CTC 1068
   Glu Val Val Trp Thr Asn Thr Pro Thr Lys Trp Asp Asn Ser Phe Leu
   320 325 330
   GAG ATC CTG TAC GGC TAC GAG TGG GAG CTG ACG AAG AGC CCT GCT GGC
   1116
   Glu Ile Leu Tyr Gly Tyr Glu Trp Glu Leu Thr Lys Ser Pro Ala Gly
       335                 340                 345
   GCT TGG CAA TAC ACC GCC AAG GAC GGC GCC GGT GCC GGC ACC ATC CCG
   1164
   Ala Trp Gln Tyr Thr Ala Lys Asp Gly Ala Gly Ala Gly Thr Ile Pro
   350                 355                 360                 365
   GAC CCG TTC GGC GGG CCA GGG CGC TCC CCG ACG ATG CTG GCC ACT GAC
   1212
   Asp Pro Phe Gly Gly Pro Gly Arg Ser Pro Thr Met Leu Ala Thr Asp
                   370                 375                 380
   CTC TCG CTG CGG GTG GAT CCG ATC TAT GAG CGG ATC ACG CGT CGC TGG
   1260
   Leu Ser Leu Arg Val Asp Pro Ile Tyr Glu Arg Ile Thr Arg Arg Trp
               385                 390                 395
   CTG GAA CAC CCC GAG GAA TTG GCC GAC GAG TTC GCC AAG GCC TGG TAC
   1308
   Leu Glu His Pro Glu Glu Leu Ala Asp Glu Phe Ala Lys Ala Trp Tyr
           400                 405                 410
   AAG CTG ATC CAC CGA GAC ATG GGT CCC GTT GCG AGA TAC CTT GGG CCG
   1356
   Lys Leu Ile His Arg Asp Met Gly Pro Val Ala Arg Tyr Leu Gly Pro
       415                 420                 425
   CTG GTC CCC AAG CAG ACC CTG CTG TGG CAG GAT CCG GTC CCT GCG GTC
   1404
   Leu Val Pro Lys Gln Thr Leu Leu Trp Gln Asp Pro Val Pro Ala Val
   430                 435                 440                 445
   AGC CAC GAC CTC GTC GGC GAA GCC GAG ATT GCC AGC CTT AAG AGC CAG
   1452
   Ser His Asp Leu Val Gly Glu Ala Glu Ile Ala Ser Leu Lys Ser Gln
                   450                 455                 460
   ATC CGG GCA TCG GGA TTG ACT GTC TCA CAG CTA GTT TCG ACC GCA TGG
   1500
   Ile Arg Ala Ser Gly Leu Thr Val Ser Gln Leu Val Ser Thr Ala Trp
               465                 470                 475
   GCG GCG GCG TCG TCG TTC CGT GGT AGC GAC AAG CGC GGC GGC GCC AAC
   1548
   Ala Ala Ala Ser Ser Phe Arg Gly Ser Asp Lys Arg Gly Gly Ala Asn
           480                 485                 490
   GGT GGT CGC ATC CGC CTG CAG CCA CAA GTC GGG TGG GAG GTC AAC GAC
   1596
   Gly Gly Arg Ile Arg Leu Gln Pro Gln Val Gly Trp Glu Val Asn Asp
       495                 500                 505
   CCC GAC GGG GAT CTG CGC AAG GTC ATT CGC ACC CTG GAA GAG ATC CAG
   1644
   Pro Asp Gly Asp Leu Arg Lys Val Ile Arg Thr Leu Glu Glu Ile Gln
   510 515 520 525
   GAG TCA TTC AAC TCC GCG GCG CCG GGG AAC ATC AAA GTG TCC TTC GCC
   1692
   Glu Ser Phe Asn Ser Ala Ala Pro Gly Asn Ile Lys Val Ser Phe Ala
   530 535 540
   GAC CTC GTC GTG CTC GGT GGC TGT GCC GCC ATA GAG AAA GCA GCA AAG
   1740
   Asp Leu Val Val Leu Gly Gly Cys Ala Ala Ile Glu Lys Ala Ala Lys
   545 550 555
   GCG GCT GGC CAC AAC ATC ACG GTG CCC TTC ACC CCG GGC CGC ACG GAT
   1788
   Ala Ala Gly His Asn Ile Thr Val Pro Phe Thr Pro Gly Arg Thr Asp
   560 565 570
   GCG TCG CAG GAA CAA ACC GAC GTG GAA TCC TTT GCC GTG CTG GAG CCC
   1836
   Ala Ser Gln Glu Gln Thr Asp Val Glu Ser Phe Ala Val Leu Glu Pro
   575 580 585
   AAG GCA GAT GGC TTC CGA AAC TAC CTC GGA AAG GGC AAC CCG TTG CCG
   1884
   Lys Ala Asp Gly Phe Arg Asn Tyr Leu Gly Lys Gly Asn Pro Leu Pro
   590 595 600 605
   GCC GAG TAC ATG CTG CTC GAC AAG GCG AAC CTG CTT ACG CTC AGT GCC
   1932
   Ala Glu Tyr Met Leu Leu Asp Lys Ala Asn Leu Leu Thr Leu Ser Ala
   610 615 620
   CCT GAG ATG ACG GTG CTG GTA GGT GGC CTG CGC GTC CTC GGC GCA AAC
   1980
   Pro Glu Met Thr Val Leu Val Gly Gly Leu Arg Val Leu Gly Ala Asn
   625 630 635
   TAC AAG CGC TTA CCG CTG GGC GTG TTC ACC GAG GCC TCC GAG TCA CTG
   2028
   Tyr Lys Arg Leu Pro Leu Gly Val Phe Thr Glu Ala Ser Glu Ser Leu
   640 645 650
   ACC AAC GAC TTC TTC GTG AAC CTG CTC GAC ATG GGT ATC ACC TGG GAG
   2076
   Thr Asn Asp Phe Phe Val Asn Leu Leu Asp Met Gly Ile Thr Trp Glu
   655 660 665
   CCC TCG CCA GCA GAT GAC GGG ACC TAC CAG GGC AAG GAT GGC AGT GGC
   2124
   Pro Ser Pro Ala Asp Asp Gly Thr Tyr Gln Gly Lys Asp Gly Ser Gly
   670 675 680 685
   AAG GTG AAG TGG ACC GGC AGC CGC GTG GAC CTG GTC TTC GGG TCC AAC
   2172
   Lys Val Lys Trp Thr Gly Ser Arg Val Asp Leu Val Phe Gly Ser Asn
                   690                 695                 700
   TCG GAG TTG CGG GCG CTT GTC GAG GTC TAT GGC GCC GAT GAC GCG CAG
   2220
   Ser Glu Leu Arg Ala Leu Val Glu Val Tyr Gly Ala Asp Asp Ala Gln
               705                 710                 715
   CCG AAG TTC GTG CAG GAC TTC GTC GCT GCC TGG GAC AAG GTG ATG AAC
   2268
   Pro Lys Phe Val Gln Asp Phe Val Ala Ala Trp Asp Lys Val Met Asn
           720                 725                 730
   CTC GAC AGG TTC GAC GTG CGC TGATTCGGGT TGATCGGCCC TGCCCGCCGA
   2319
   Leu Asp Arg Phe Asp Val Arg
       735                 740
   TCAACCACAA CC
   2331

### (2) INFORMATION FOR SEQ ID NO:21:

(i) SEQUENCE CHARACTERISTICS:
   (A) LENGTH: 740 amino acids
   (B) TYPE: amino acid
   (D) TOPOLOGY: linear
(ii) MOLECULE TYPE: protein
(xi) SEQUENCE DESCRIPTION: SEQ ID NO:21:
   Val Pro Glu Gln His Pro Pro Ile Thr Glu Thr Thr Thr Gly Ala Ala
    1               5                  10                  15
   Ser Asn Gly Cys Pro Val Val Gly His Met Lys Tyr Pro Val Glu Gly
               20                  25                  30
   Gly Gly Asn Gln Asp Trp Trp Pro Asn Arg Leu Asn Leu Lys Val Leu
           35                  40                  45
   His Gln Asn Pro Ala Val Ala Asp Pro Met Gly Ala Ala Phe Asp Tyr
       50                  55                  60
   Ala Ala Glu Val Ala Thr Ile Asp Val Asp Ala Leu Thr Arg Asp Ile
   65                  70                  75                  80
   Glu Glu Val Met Thr Thr Ser Gln Pro Trp Trp Pro Ala Asp Tyr Gly
                   85                  90                  95
   His Tyr Gly Pro Leu Phe Ile Arg Met Ala Trp His Ala Ala Gly Thr
               100                 105                 110
   Tyr Arg Ile His Asp Gly Arg Gly Gly Ala Gly Gly Gly Met Gln Arg
           115                 120                 125
   Phe Ala Pro Leu Asn Ser Trp Pro Asp Asn Ala Ser Leu Asp Lys Ala
       130                 135                 140
   Arg Arg Leu Leu Trp Pro Val Lys Lys Lys Tyr Gly Lys Lys Leu Ser
   145                 150                 155                 160
   Trp Ala Asp Leu Ile Val Phe Ala Gly Asn Cys Ala Leu Glu Ser Met
                   165                 170                 175
   Gly Phe Lys Thr Phe Gly Phe Gly Phe Gly Arg Val Asp Gln Trp Glu
               180                 185                 190
   Pro Asp Glu Val Tyr Trp Gly Lys Glu Ala Thr Trp Leu Gly Asp Glu
           195                 200                 205
   Arg Tyr Ser Gly Lys Arg Asp Leu Glu Asn Pro Leu Ala Ala Val Gln
       210                 215                 220
   Met Gly Leu Ile Tyr Val Asn Pro Glu Gly Pro Asn Gly Asn Pro Asp
   225                 230                 235                 240
   Pro Met Ala Ala Ala Val Asp Ile Arg Glu Thr Phe Arg Arg Met Ala
                   245                 250                 255
   Met Asn Asp Val Glu Thr Ala Ala Leu Ile Val Gly Gly His Thr Phe
               260                 265                 270
   Gly Lys Thr His Gly Ala Gly Pro Ala Asp Leu Val Gly Pro Glu Pro
           275                 280                 285
   Glu Ala Ala Pro Leu Glu Gln Met Gly Leu Gly Trp Lys Ser Ser Tyr
       290                 295                 300
   Gly Thr Gly Thr Gly Lys Asp Ala Ile Thr Ser Gly Ile Glu Val Val
   305                 310                 315                 320
   Trp Thr Asn Thr Pro Thr Lys Trp Asp Asn Ser Phe Leu Glu Ile Leu
                   325                 330                 335
   Tyr Gly Tyr Glu Trp Glu Leu Thr Lys Ser Pro Ala Gly Ala Trp Gln
               340                 345                 350
   Tyr Thr Ala Lys Asp Gly Ala Gly Ala Gly Thr Ile Pro Asp Pro Phe
           355                 360                 365
   Gly Gly Pro Gly Arg Ser Pro Thr Met Leu Ala Thr Asp Leu Ser Leu
       370                 375                 380
   Arg Val Asp Pro Ile Tyr Glu Arg Ile Thr Arg Arg Trp Leu Glu His
   385                 390                 395                 400
   Pro Glu Glu Leu Ala Asp Glu Phe Ala Lys Ala Trp Tyr Lys Leu Ile
                   405                 410                 415
   His Arg Asp Met Gly Pro Val Ala Arg Tyr Leu Gly Pro Leu Val Pro
               420                 425                 430
   Lys Gln Thr Leu Leu Trp Gln Asp Pro Val Pro Ala Val Ser His Asp
           435                 440                 445
   Leu Val Gly Glu Ala Glu Ile Ala Ser Leu Lys Ser Gln Ile Arg Ala
       450                 455                 460
   Ser Gly Leu Thr Val Ser Gln Leu Val Ser Thr Ala Trp Ala Ala Ala
   465                 470                 475                 480
   Ser Ser Phe Arg Gly Ser Asp Lys Arg Gly Gly Ala Asn Gly Gly Arg
                   485                 490                 495
   Ile Arg Leu Gln Pro Gln Val Gly Trp Glu Val Asn Asp Pro Asp Gly
               500                 505                 510
   Asp Leu Arg Lys Val Ile Arg Thr Leu Glu Glu Ile Gln Glu Ser Phe
           515                 520                 525
   Asn Ser Ala Ala Pro Gly Asn Ile Lys Val Ser Phe Ala Asp Leu Val
       530                 535                 540
   Val Leu Gly Gly Cys Ala Ala Ile Glu Lys Ala Ala Lys Ala Ala Gly
   545                 550                 555                 560
   His Asn Ile Thr Val Pro Phe Thr Pro Gly Arg Thr Asp Ala Ser Gln
                   565                 570                 575
   Glu Gln Thr Asp Val Glu Ser Phe Ala Val Leu Glu Pro Lys Ala Asp
               580                 585                 590
   Gly Phe Arg Asn Tyr Leu Gly Lys Gly Asn Pro Leu Pro Ala Glu Tyr
           595                 600                 605
   Met Leu Leu Asp Lys Ala Asn Leu Leu Thr Leu Ser Ala Pro Glu Met
       610                 615                 620
   Thr Val Leu Val Gly Gly Leu Arg Val Leu Gly Ala Asn Tyr Lys Arg
   625                 630                 635                 640
   Leu Pro Leu Gly Val Phe Thr Glu Ala Ser Glu Ser Leu Thr Asn Asp
                   645                 650                 655
   Phe Phe Val Asn Leu Leu Asp Met Gly Ile Thr Trp Glu Pro Ser Pro
               660                 665                 670
   Ala Asp Asp Gly Thr Tyr Gln Gly Lys Asp Gly Ser Gly Lys Val Lys
           675                 680                 685
   Trp Thr Gly Ser Arg Val Asp Leu Val Phe Gly Ser Asn Ser Glu Leu
       690                 695                 700
   Arg Ala Leu Val Glu Val Tyr Gly Ala Asp Asp Ala Gln Pro Lys Phe
   705                 710                 715                 720
   Val Gln Asp Phe Val Ala Ala Trp Asp Lys Val Met Asn Leu Asp Arg
                   725                 730                 735
   Phe Asp Val Arg
               740

### (2) INFORMATION FOR SEQ ID NO:22:

(i) SEQUENCE CHARACTERISTICS:
   (A) LENGTH: 15 base pairs
   (B) TYPE: nucleic acid
   (C) STRANDEDNESS: single
   (D) TOPOLOGY: linear
(ii) MOLECULE TYPE: DNA
(xi) SEQUENCE DESCRIPTION: SEQ ID NO:22:
   GACGCNNNNN NNNNN
   15

## Description

### Background of the Invention

Despite more than a century of research since the discovery of *Mycobacterium tuberculosis,* the aetiological agent of tuberculosis, this disease remains one of the major causes of human morbidity and mortality. There are an estimated 3 million deaths annually attributable to tuberculosis (see, D. Snider, *Rev. Inf. Dis*., S335 (1989)), and although the majority of these are in developing countries, the disease is assuming renewed importance in the West due to the increasing number of homeless people and the impact the AIDS epidemic (see, R.E. Chaisson et al., *Am. Res. Resp. Dis.,* 23, 56 (1987); D. E. Snider, Jr. et al., *New Engl. J. Med*., 326, 703(1992); M.A. Fischl et al., *Ann*. *Int*. *Med*., 117, 177 (1992) and ibid. at 184.

Isonicotinic acid hydrazide or isoniazid (INH) has been used in the treatment of tuberculosis for the last forty years due to its exquisite potency against the members of the "tuberculosis" groups - *Mycobacterium tuberculosis*, *M. bovis* and *M. africanum* (G. Middlebrook, *Am. Rev. Tuberc.,* 69, 471(1952) and J. Youatt, *Am. Rev. Resp. Dis.,* 99, 729 (1969)). Neither the precise target of the drug, nor its made of action are known, but INH treatment results in the perturbation of several metabolic pathways of the bacterium. However, shortly after its introduction, INH-resistant isolates of *Mycobacterium tuberculosis* emerged. See M.L. Pearson et al., *Ann. Int. Med*., 117, 191 (1992) and S.W. Dooley et al., *Ann. Int. Med*., 117, 257 (1992).

Several investigators have associated the toxicity of INH for mycobacteria with endogenous catalase activity. See, for example, "Isonicotinic acid hydrazide," in F. E. Hahn, *Mechanism of Action of Antibacterial Agents*, Springer-Verlag (1979) at pages 98-119. This relationship was strengthened by a recent report by Ying Zhang and colleagues in *Nature*, 358, 591 (1992) which described the restoration of INH susceptibility in an INH resistant *Mycobacterium smegmatis* strain after transformation using the catalase-peroxide (*katG*) gene from an INH sensitive *M. tuberculosis* strain. In a follow-up study, Zhang and colleagues in *Molec. Microbiol*., 8, 521 (1993) demonstrated the restoration of INH susceptibility in INH resistant *M*. *tuberculosis* strains after transformation by the functional *katG* gene. As reported by B. Heym et al., *J. Bacterial*., 175, 4255 (1993), the *katG* gene encodes for a 80,000-dalton protein. The deletions and mutations of the *katG* gene of INH-resistant *M. tuberculosis* strains isolated from Finnish patients has been studied (Marttila et al., *Antimicrobial Agents and Chemotherapy.,* 40, 2187 (1996)). Cockerill et al., (*J. Infec. Dis*., 171, 240 (1995)) disclose a comprehensive analysis of the *katG* gene in multiple strains of *M. tuberculosis* with a wide range of isoniazid resistance phenotypes.

A conclusive diagnosis of tuberculosis depends on the isolation and identification of the etiologic agent, *Mycobacterium tuberculosis,* which generally requires 3-8 weeks. Design of an appropriate therapeutic regimen depends on the results of subsequent antituberculosis susceptibility testing by the agar dilution method and produces additional delays of 3-6 weeks (Roberts et al., "Mycobacerium" in *Manual of Clincial Microbiology,* 5th Ed.; A. Balows et al., Eds.; American Society for Microbiology: Washington; pp. 304-399 (1991). Identifcaiton and drug resistance testing can now also be accomplished more quickly by using the BACTEC radiometric method. (Tenover et al., *J. Clin. Microbiol*., 31, 767-779 (1993) and Huebner et al., *J. Clin. Microbiol*., 31, 771-775 (1993). Acid fast bacilli are detected in the BACTEC bottle, and and identification is made using a nucleic acid hybridization technique on the BACTEC-derived growth. Drug susceptibility testing is then conducted using the same BACTEC growth to inoculate fresh BACTEC bottles containing various antituberculosis drugs. This procedure reduces the time needed to generate a complete analysis, but the total time required to report susceptibility results for MTB is typically in excess of 20 days. The need to minimize the transmission of newly identified drug resistant strains of MTB requires the development of much more rapid identification procedures.

The rapid detection of *M. tuberculosis* directly from clinical samples has been possible recently by virtue of the availability of polymerase chain reaction (PCR) and the recognition of diagnostic sequences amplified by the appropriate primers. The ability to conduct PCR analyses depends on having a high enough gene or gene product concentration so that the molecular tools work efficiently even when the organism numbers are low. Thus, the most efficient molecular assays used to detect *M. tuberculosis* depend on the IS6110 insertion sequence(about 10 copies) or the 16S ribosomal RNA (thousands of copies). See, respectively, K.D. Eisenach et al., *J. Infect. Dis.,* 61, 997 (1990) and N. Miller et al., *Abstracts ASM*, Atlanta, GA (1993) at page 177. However, these methods do not provide any information regarding the drug-resistance phenotype of the *M. tuberculosis* strain.

There have been several studies regarding the analysis of the *M*. *tuberculosis katG* gene. A method demonstrating that wild-type *M. tuberculosis* can be distinguished from M. *tuberculosis* mutants, and *M. tuberculosis* mutants can be distinguished from one another is disclosed in WO-A-96 15267 (Dahlberg, et al.). Temesgen et al., (*Molec. Cellul. Probes,* 11, 59 (1997)) evaluate the utility of the PCR-SSCP method for detecting the *katG* R463L mutation by comparing it with results obtained using the PCR-*Msp*I RFLP method.

Recently, B. Heym et al. (PCT WO 93/22454) disclose the use of polymerase chain reaction to amplify portions of the *katG* gene of putative resistant strains. The PCR products were evaluated by single-strand conformation polymorphism (SSCP) analysis, wherein abnormal strand motility on a gel is associated with mutational events in the gene. For example, in five strains, a single base difference was found in a 200 bp sequence, a G to T transversion at position 3360. This difference would result in the substitution of Arg-461 by Leu. However, carrying out SSCP on a given clinical sample can be a laborious procedure that requires sequencing to confirm whether mutations or deletions predictive of drug resistance are in fact present in the target gene.

There is a continuing need in the art to develop a simple test permitting the rapid identification of *M. tuberculosis* and its drug-resistance phenotype.

### Summary of the Invention

The present invention provides a method to selectively identify M. tuberculosis in a sample containing DNA.

Said method may be combined with a method to rapidly identify strains of *M. tuberculosis* which are resistant to isoniazid (INH). The method is based on our discovery that certain mutations in the *katG* gene of *M. tuberculosis* which confer INH resistance coincidentally result in the addition or deletion of restriction sites, which are recognized by various restriction enzymes. For example, the wild-type (WT) *katG* gene of *M. tuberculosis* contains an *Nci*I-*Msp*I restriction site spanning codon 463, which site is absent in the corresponding codon 463 in a number of INH resistant strains due to a single base mutation in that codon. An *Nci*I-*Msp*I restriction site is a site cleaved by both *Nci*I and *Msp*I. This site is represented by the nucleotide sequence CCGGG (see Table 1).

Alternatively, or in addition, some INH resistant strains have a single base mutation in codon 315 in the *katG* gene that produces a new *Msp*I restriction site associated with the corresponding codon 315 in the INH resistant strain. Some INH resistant strains have a single base mutation at codon 337 that results in the deletion of a *Rsa*I restriction site otherwise present at the corresponding position in the WT gene; and some have a single base mutation at codon 264 that eliminates a *Cfo*I restriction site. These mutations may be present singly or in combination in INH resistant M. *tuberculosis* strains.

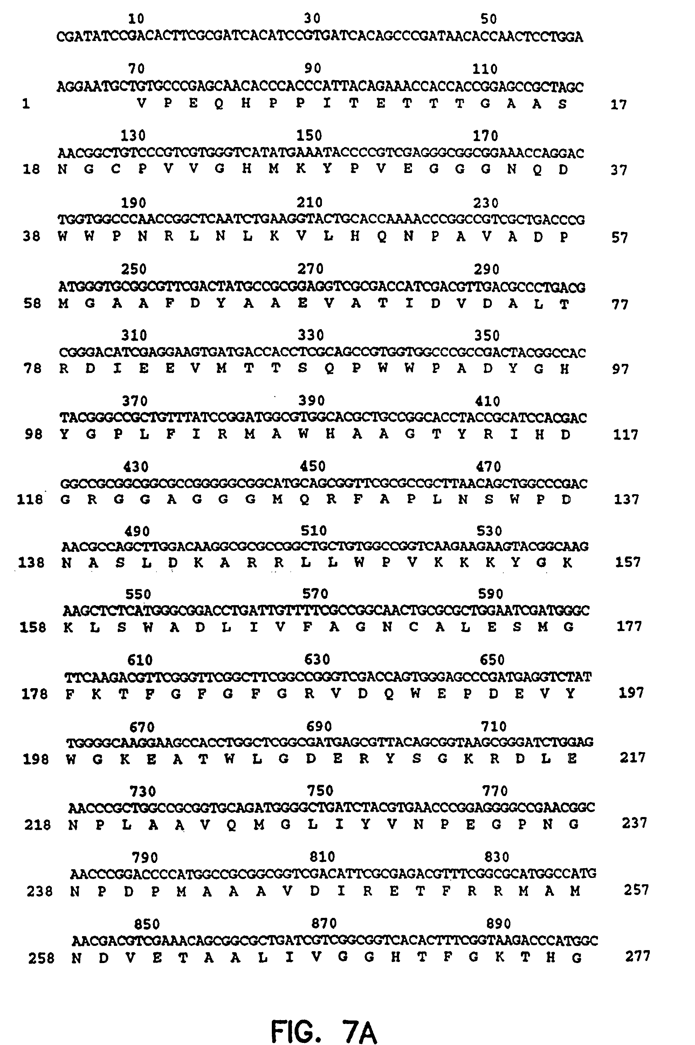

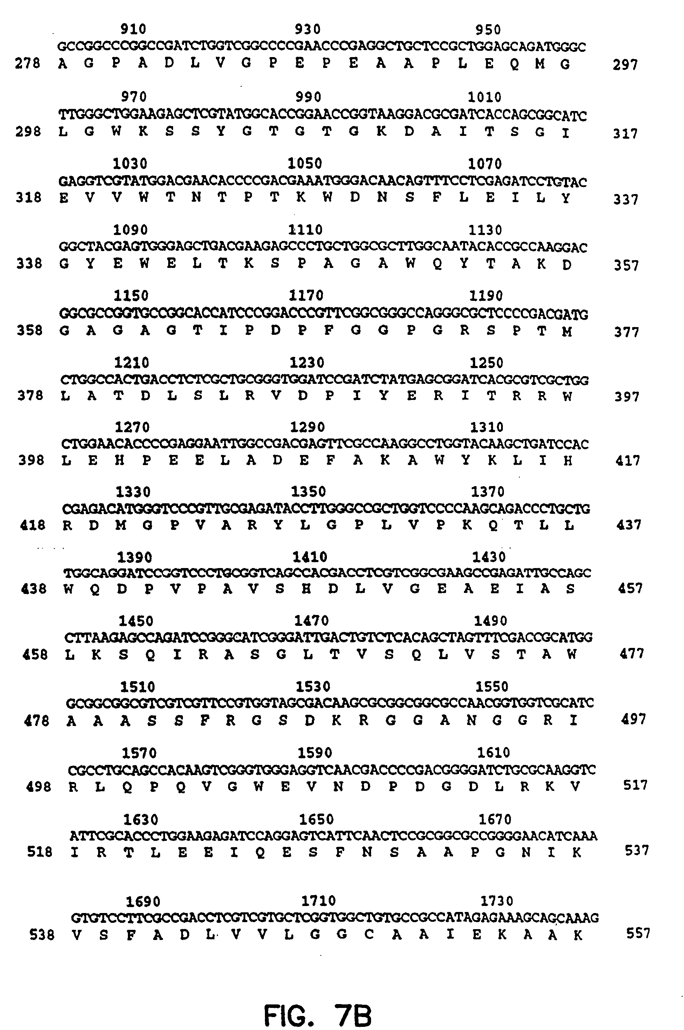

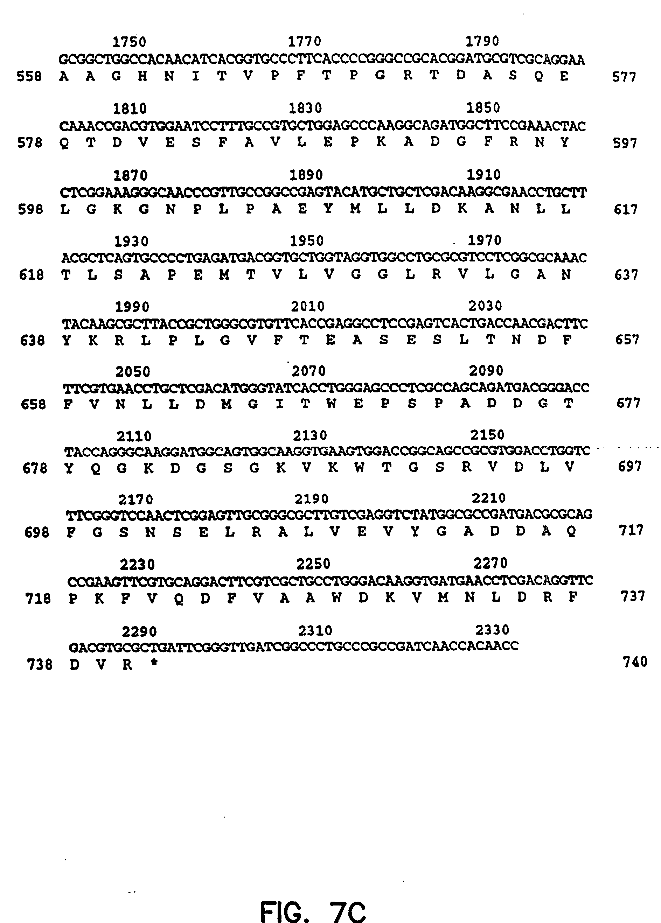

When used in reference to nucleotide position, codon position or restriction site position, the term "corresponding" is defined to mean the same absolute location on two different M. *tuberculosis katG* genes, wherein absolute location is defined by the numbering system used in Figure 7 (SEQ ID NO:20). For example, a wild-type codon 463 represented by CGG at nucleotide positions 1456-1458 on a wild-type *katG* gene of *M. tuberculosis* and a mutant codon 463 represented by CTG at the same nucleotide positions 1456-1458 on *a katG* gene of an INH resistant strain of *M. tuberculosis* are considered to be corresponding codons.

The determination of whether one or more of these identifying mutations in the *katG* gene are present in a strain of *M. tuberculosis* can be made by employing the techniques of restriction fragment length polymorphism (RFLP) analysis. Therefore, in an embodiment directed to the identification of a mutation in codon 463 that is associated with INH resistance, the present assay comprises the steps of:
(a) amplifying a portion of the *katG* gene of an *M. tuberculosis* isolate to yield a detectable amount of DNA comprising the nucleotide position occupied by base 1457 of the *M. tuberculosis katG* gene consensus sequence depicted in Figure 7 (SEQ ID NO:20); and
(b) determining whether an *Nci*I-*Msp*I restriction site is absent in codon 463 of said *katG* gene, wherein said absence is indicative of an INH resistant strain of *M. tuberculosis*.

The RFLP technique involves cleaving the DNA with a restriction endonuclease which cleaves at an *Nci*I-*Msp*I restriction site to yield at least one DNA fragment and determining whether the number and location of the fragments is indicative of the absence of an *Nci*I-*Msp*I restriction site in codon 463 of said *katG* gene, wherein said absence is indicative of an INH resistant strain of *M. tuberculosis,* preferably by employing the techniques of gel electrophoresis.

If the amplified DNA of step (a) contains no *Nci*I-*Msp*I restriction sites, then the DNA fragment yielded in step (b) will be identical to the amplified DNA of step (a). This can occur where the portion of the *katG* gene amplified in step (a) is from an INH resistant strain of *M. tuberculosis* having a mutation in codon 463 that removes the *Nci*I-*Msp*I restriction site spanning that codon in the wild-type *katG* gene, and having no other additional *Nci*I-*Msp*I restriction sites.

In order for the amplified DNA to yield a meaningful RFLP pattern, the portion of the *katG* gene amplified in step (a) will be of sufficient length to produce fragments of sufficient length to visualize using gel electrophoresis. In the above-described embodiment, for example, the portion amplified will contain a sufficient number of bases to either side (5' or 3') of codon 463 such that cleavage at a site spanning that codon will yield fragments that can be visualized using gel electrophoresis.

In another embodiment of the invention directed to the additional identification of a mutation in codon 315 associated with INH resistance, the amplified DNA of step (a) further comprises at least one *Msp*I restriction site and the nucleotide position occupied by base 1013 (Figure 7, SEQ ID NO:20), and the determination made in step (b) further includes whether an *Msp*I restriction site associated with codon 315 is present, wherein said presence is indicative of an INH resistant strain. For example, RFLP can also be employed to determine whether the number and location of the fragments is indicative of the codon 315 *Msp*I restriction site. Preferably, the portion of the *katG* locus which is amplified is a minor portion of the entire *katG* gene, i.e., less than 1500 base pair, more preferably less than 1000 base pair, and is isolated and amplified by polymerase chain reaction, as described hereinbelow. The term "location" refers to the Rf (relative electrophoretic mobility) of a given fragment on the gel.

The pattern of fragments produced on a gel by electrophoresis of a restriction digest of an amplified portion of the *katG* gene of an *M*. *tuberculosis* strain of interest, such as an INH resistant strain, is preferably compared to the pattern produced in a digest of an equivalent portion of the *katG* gene of a wild-type (WT) control strain of *M*. *tuberculosis,* which strain is INH sensitive. The term "equivalent" is defined herein to mean that any two portions of the *katG* gene would comprise the same number and location of restriction sites being analyzed (e.g., sites recognized by *Cfo*I, *Rsa*I, *Msp*I, and/or *Nci*I) if the portions both were selected from a portion of the DNA of SEQ ID NO: 20 (i.e, if there were no mutations altering the number of restriction sites of the type being analyzed), and that the portions do not differ in size before cleavage to the extent that the number of fragments obtained cannot be compared following side-by-side gel electrophoresis and visualization of the resultant fragments, as described hereinbelow. For example, the control *kat*G DNA can correspond to an equivalent portion of SEQ ID NO:20 (Figure 7, upper sequence) comprising one or more of the codons of interest (e.g., codons 315 or 463) and their associated restriction sites. As discussed below, such a portion of DNA can be derived from strain H37Rv MC. A positive control corresponding to DNA fragments derived from a known INH resistant strain may also be used.

In the embodiment of the assay of the invention directed to the determination of the presence or absence of a *Nci*I*-Msp*I restriction site associated with codon 463, gel electrophoresis is employed to compare the number and location of the DNA fragments to the number and location of DNA fragments derived from cleavage of DNA derived from an equivalent portion of the *katG* gene wherein the *Nci*I-*Msp*I restriction site at codon 463 is present, wherein a determination of the absence of the restriction site at codon 463 in the *katG* gene is indicative of an INH resistant strain of *M. tuberculosis*. Preferably, the control DNA sequence of the portion of the *katG* gene wherein the codon 463 restriction site is present corresponds to a portion of SEQ ID NO:20 (Figure 7, upper sequence). For example, the control DNA may contain five *Nci*I-*Msp*I restriction sites in each DNA molecule prior to cleavage, and the DNA of step (a), which is derived from an INH resistant strain, may contain four *Nci*I-*Msp*I restriction sites in each DNA molecule prior to cleavage. The assay also preferably includes positive control DNA fragments derived from an INH resistant strain which does not include the codon 463 *Nci*I-*Msp*I restriction site in the *katG* gene.

The present invention provides method for selectively detecting *M. tuberculosis* in a DNA sample, wherein the DNA is amplified to generate a detectable amount of amplified DNA comprising a *katG* DNA fragment which consists of base 904 through base 1523 of the *M. tuberculosis katG* gene. The generation of this *katG* DNA fragment is indicative of the presence of *M. tuberculosis* in the sample. Preferably, the DNA sample is a human biological tissue or fluid, more preferably a human biological fluid. The DNA sample is most preferably human sputum. Advantageously, the method of the invention can be performed on clinical human sputum samples with minimal pretreatment of the clinical sample.

In a preferred embodiment of the *M. tuberculosis* detection method, the *katG* DNA fragment has a restriction site that comprises either a G or a C at the nucleotide position occupied by base 1013 in codon 315 of the *M. tuberculosis katG* gene as depicted in Figure 7 (SEQ ID NO: 20), and the method further comprises contacting the *katG* DNA fragment with a restriction endonuclease, preferably *Msp*I, that cleaves either at the restriction site comprising a G at the nucleotide position occupied by base 1013 of codon 315, or at the restriction site comprising a C at the nucleotide position occupied by base 1013 of codon 315, but not both, yielding at least one cleaved fragment. The at least one cleaved fragment is electrophoresed to yield an electrophoretic mobility pattern comprising the at least one cleaved fragment, and the mobility pattern is analyzed to selectively detect the presence of *M*. *tuberculosis in* the sample. Preferably, restriction fragment length polymorphism (RFLP) analysis is used to analyze the electrophoretic mobility pattern generated by gel electrophoresis of the cleaved fragments to detect *M. tuberculosis.*

In another embodiment of the *M. tuberculosis* detection method, *M. tuberculosis* is selectively detected in a DNA sample by:
(a) amplifying the DNA to generate a detectable amount of amplified DNA comprising a *katG* DNA fragment comprising base 904 through base 1523 of the *M. tuberculosis katG* gene, wherein the *katG* DNA fragment further comprises a restriction site comprising either a G or a C at the nucleotide position occupied by base 1013 in codon 315 of the *M. tuberculosis katG* gene as depicted in Figure 7 (SEQ ID NO: 20);
(b) contacting the *katG* DNA fragment with a restriction endonuclease, preferably *Msp*I, that cleaves either at said restriction site comprising a G at the nucleotide position occupied by base 1013 of codon 315, or at said restriction site comprising a C at the nucleotide position occupied by base 1013 of codon 315, but not at both of said restriction sites, to yield at least one cleaved fragment;
(c) electrophoresing, preferably using gel electrophoresis, the at least one cleaved fragment to yield an electrophoretic mobility pattern comprising the at least one cleaved fragment; and
(d) analyzing the mobility pattern, preferably using RFLP, to selectively detect the presence of *M. tuberculosis* in the sample.

In a further embodiment of the *M. tuberculosis* detection method, *M. tuberculosis* is selectively detected a sample containing DNA by:
(a) amplifying the DNA to generate a detectable amount of amplified DNA comprising a *katG* DNA fragment comprising base 904 through base 1523 of the *M. tuberculosis katG* gene as depicted in Figure 7 (SEQ ID NO: 20);
(b) contacting the *katG* DNA fragment with a restriction endonuclease, preferably *Msp*I, that cleaves at C/CGG to yield at least one cleaved fragment;
(c) electrophoresing, preferably using gel electrophoresis, the at least one cleaved fragment to yield a mobility pattern comprising the at least one cleaved fragment; and
(d) analyzing the mobility pattern, preferably using RFLP, to selectively detect the presence of *M. tuberculosis* in the sample.

In a particularly preferred embodiment, the *M. tuberculosis* detection method further comprises determining whether or not the *katG* DNA fragment has a S315T mutation, preferably using a restriction digest followed by gel electrophoresis of the digested DNA and RFLP analysis of the electrophoretic mobility patterns, wherein the presence of a S315T mutation is indicative of an INH-resistant strain of *M. tuberculosis*.

Oligonucleotides and subunits thereof useful in pairs as primers to initiate the polymerase chain reaction (PCR) are also disclose. Subunits of at least seven bases in length are preferred. PCR is useful both to amplify *katG* DNA so as to prepare both the target DNA of step (a) of the present process, as well as the DNA which is used to prepare the control digest.

Also disclosed is an isolated, purified DNA represented by the consensus sequence derived for the M. *tuberculosis katG* gene. This DNA was found to occur in nature as the *katG* gene of *M*. *tuberculosis* strain H37Rv MC, as maintained at the Mayo Clinic, and is also referred to as the wild-type (WT) DNA. Also disclosed is an isolated, purified DNA encoding the consensus amino acid sequence encoded by the consensus wild-type *katG* DNA, as well as DNA sequences that differ in sequence but which also encode this amino acid sequence (a consensus catalase peroxidase polypeptide) and can be employed to provide the isolated, purified polypeptide represented by the consensus amino acid sequence, which polypeptide is also said disclosed.

The polypeptide said can be prepared by expression in transformed host cells, such as bacteria, yeast, plant, or insect cells transformed with the DNA sequences of the present invention, operatively linked to regulatory regions functional in the transformed host cells. The polypeptide can be used as a standard *M. tuberculosis* catalase peroxidase, to correlate enzymatic activity (relative level, loss and restoration), with INH modification and degradation and drug resistance in *M. tuberculosis*.

Also disclosed is a kit comprising, separately packaged in association:
(a) a pair of oligonucleotide primers selected so as to amplify a portion of the DNA of the *M. tuberculosis katG* gene comprising base 1457 in codon 463 or base 1013 in codon 315, as depicted in Figure 7 (SEQ ID NO:20); and
(b) an amount of a restriction endonuclease such as *Msp*I, effective to cleave the amplified portion of said DNA at a restriction site comprising said base 1457 or said base 1013.

The present kits will also preferably comprise instruction means for carrying out the present assay, i.e., a printed package insert, tag or label, or an audio or video tape. The present kits will also preferably comprise a control DNA digest prepared by amplifying a portion of the consensus DNA of SEQ ID NO: 20 (Figure 7), that is equivalent to the portion defined and amplified by the pair of primers, followed by digestion of the DNA with a suitable restriction endonuclease such as *Msp*I.

The present invention is exemplified by the use of *Nci*I, *Msp*I, *Cfo*I*,* and *Rsa*I digestions, with the use of *Msp*I digestion being preferred; however, any restriction endonuclease having a restriction site spanning all or a portion of codon 463, codon 315, codon 337, or codon 264, which portion contains the site of the single base mutation associated with INH resistance as identified in Table 2, may be used, as desired. For example, the restriction endonucleases listed in Table 1 can be employed. Particularly preferred are restriction endonucleases having a restriction site that contains the position occupied by base 1457 in codon 463, or base 1013 in codon 315, as depicted in Figure 7 (SEQ ID NO:20).

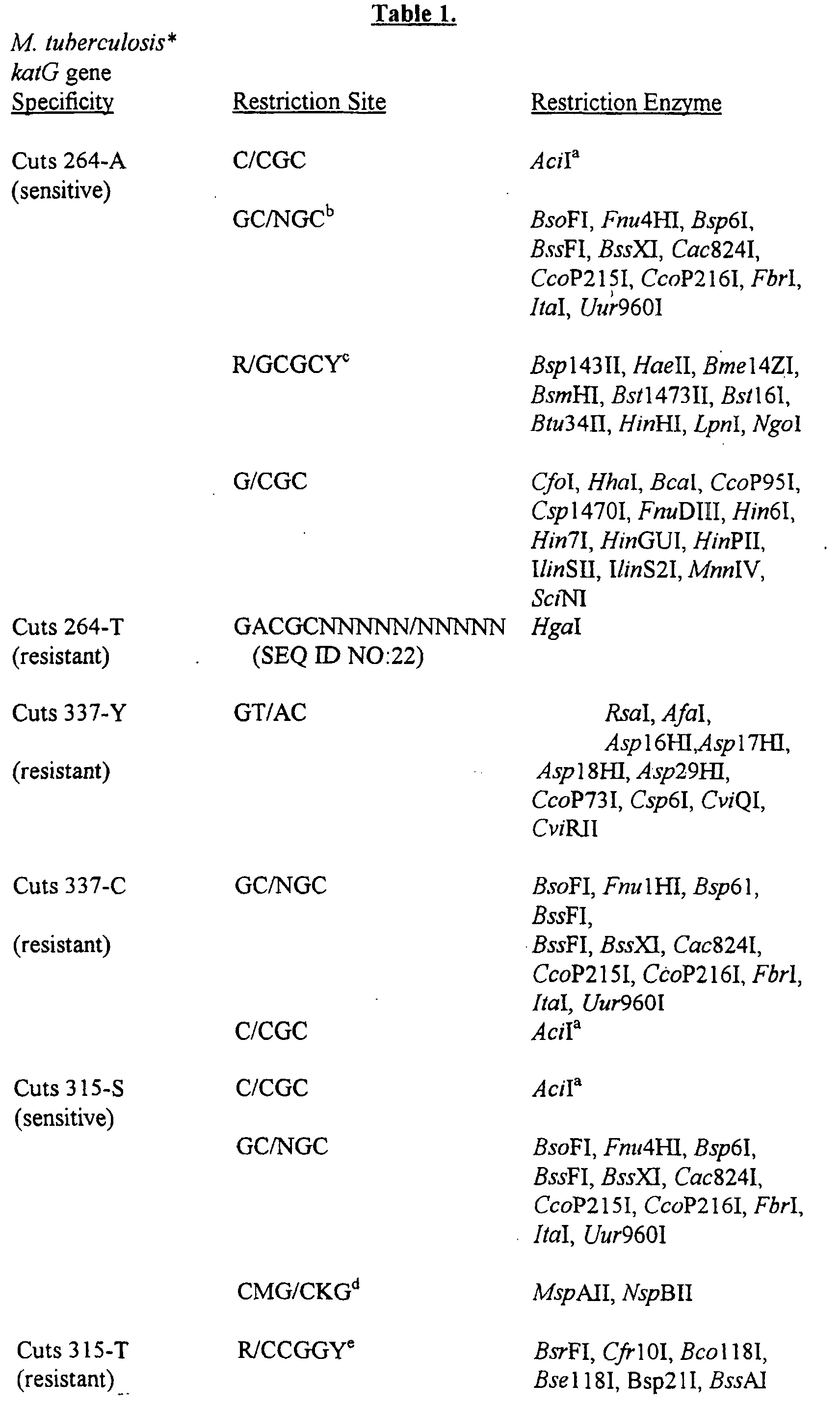

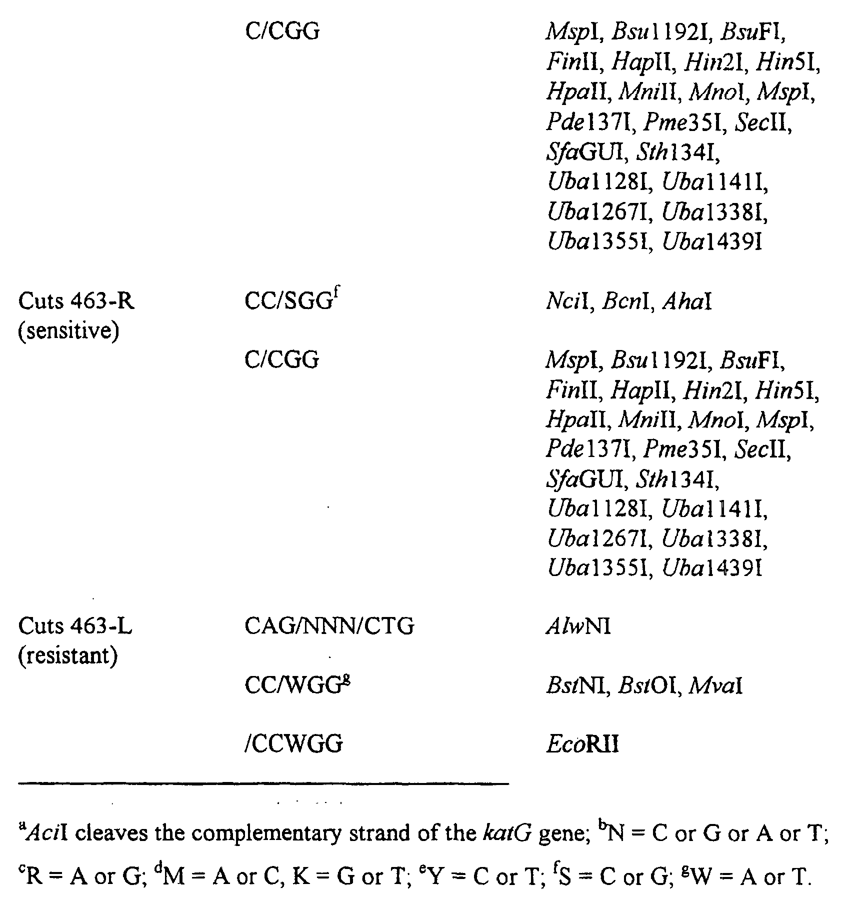

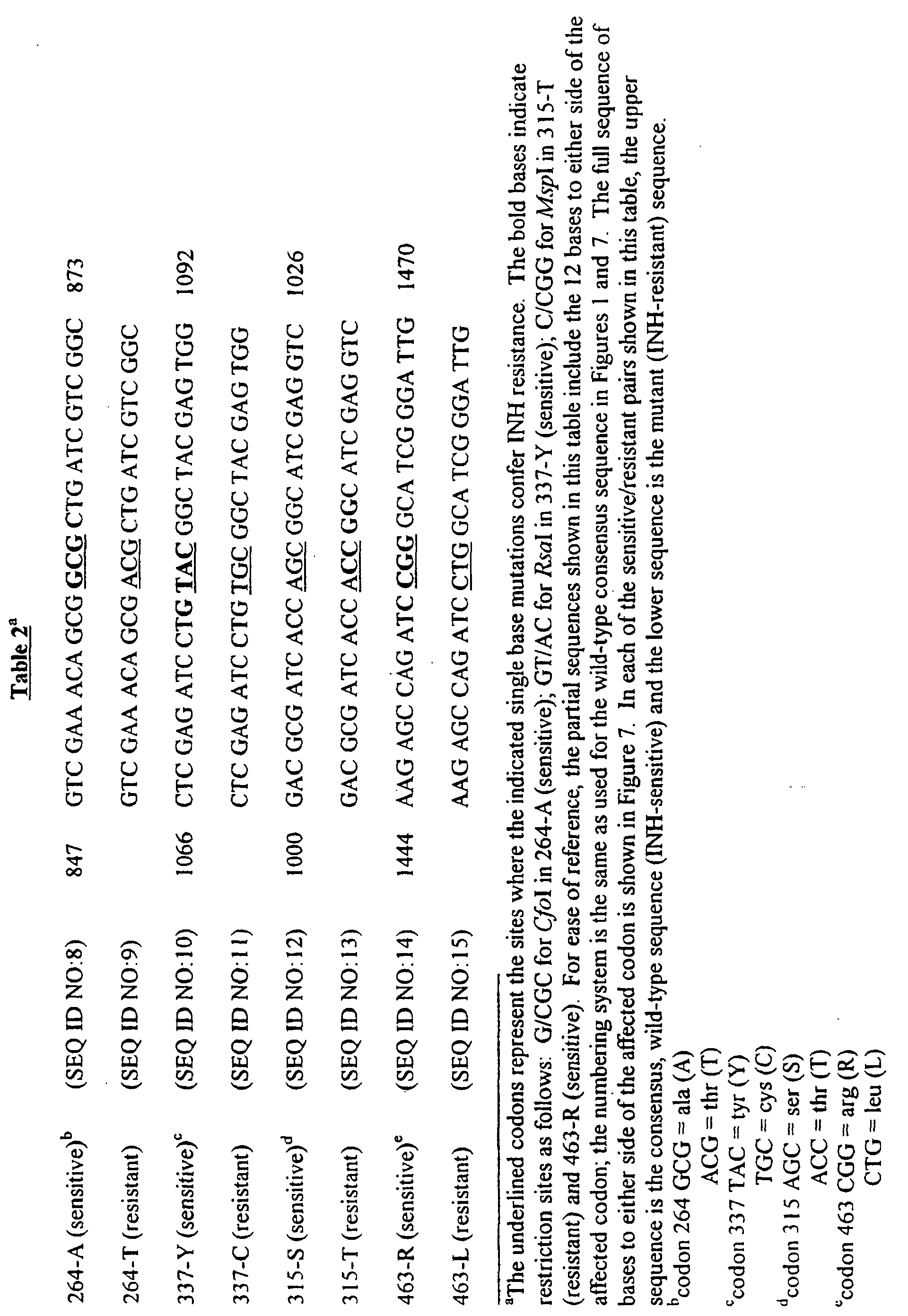

### Brief Description of the Drawings

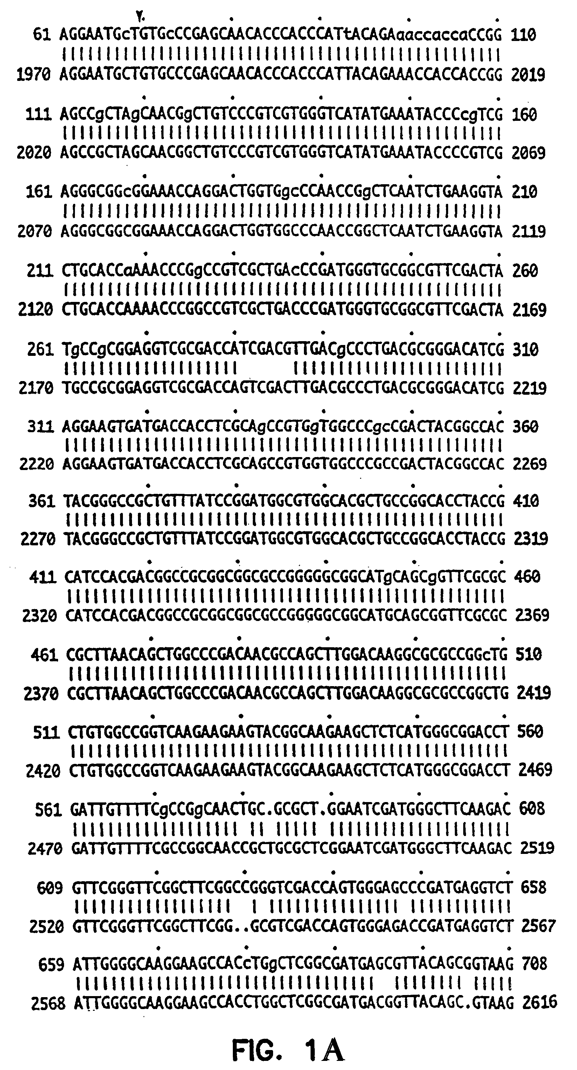

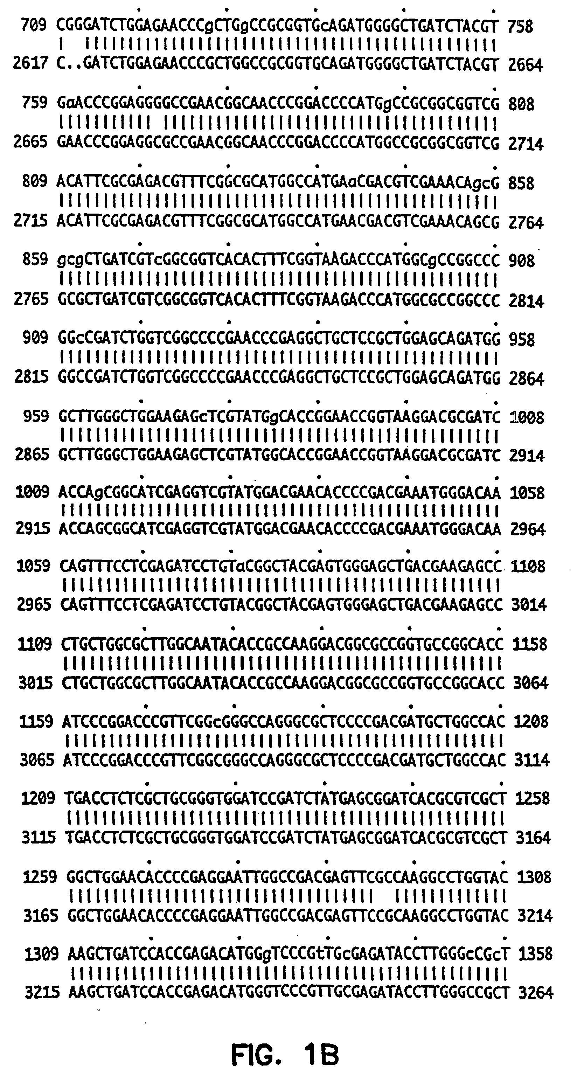

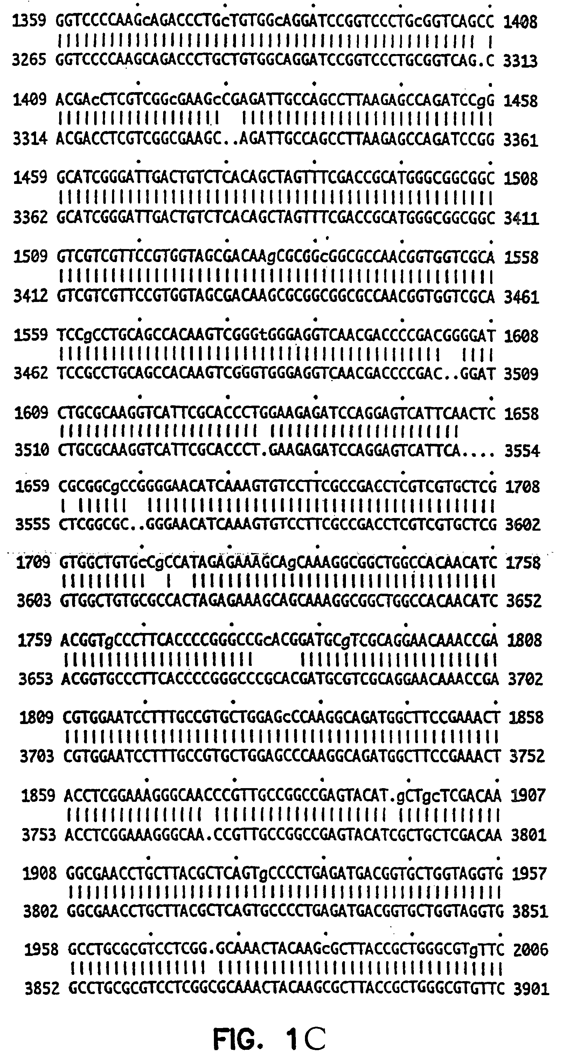

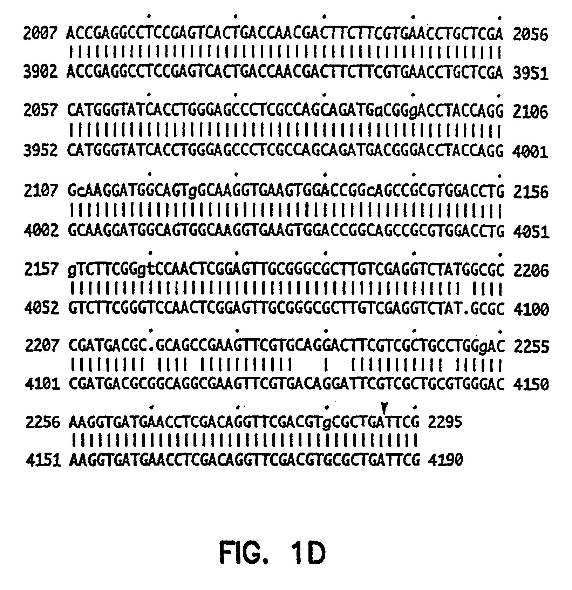

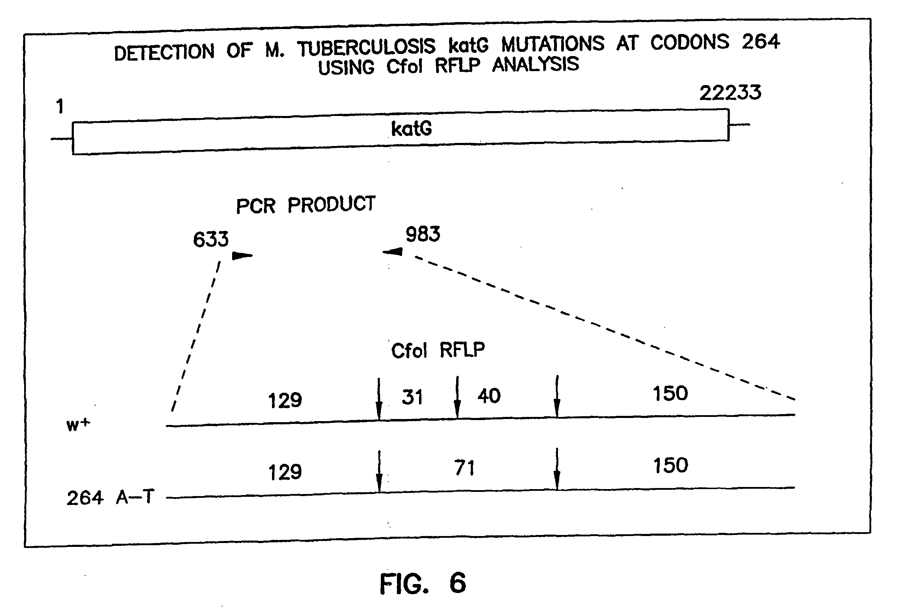

Figure 1, panels A-D, depicts the consensus, wild-type DNA sequence of the *M tuberculosis katG* gene as the upper of the pair of sequences (61-2295) (SEQ ID NO: 1). This DNA sequence data has been submitted to Gen Bank and has been assigned accession number U06262. The lower of the pair of sequences depicts nucleotide sequence 1970-4190 of the *Kpn*I fragment bearing the *katG* gene as depicted in Figure 6 of Institute Pasteur et al. (published PCT application WO 93/22454). This sequence (SEQ ID NO: 2) has been deposited in the EMBL data library under accession number X68081 (Gen Bank X68081. gb_ba). Dots (.) above the sequence mark every tenth base. The upper sequence is in lower case in areas where variation in the sequence among isolates described hereinbelow and the consensus sequence was found. The arrow before position 70 and after position 2291 of the upper sequence indicate the coding sequence of the *katG* gene.
Figure 2 depicts the *katG* amino acid consensus sequence derived from 15 strains of *M*. *tuberculosis* (SEQ ID NO: 7).
Figure 3 schematically depicts the *Ncil* restriction sites for the part B amplicon of *katG*. The (*) depicts the site of the Arg→Leu mutation which is found in some INH resistant *M. tuberculosis* strains.
Figure 4 depicts the results of a gel electrophoresis of the *Nci*I digest of the part B amplicon of 14 strains of *M. tuberculosis* (1-14).
Figure 5 schematically depicts *Msp*I and *Rsα*I restriction sites and resulting RFLP fragments for a portion of the *M. tuberculosis katG* gene. For *Msp*I, restriction maps for wild-type (W+), single (315 Ser→Thr or 463 Arg→Leu) mutants and the double (315 Ser→Thr and 463 Arg→Leu) mutant are shown. For *Rs*αI, restriction maps for wild-type (W+) and the 337 W→C mutant are shown.
Figure 6 schematically depicts *Cfo*I restriction sites and resulting RFLP fragments for a portion of the M. *tuberculosis katG* gene. Restriction maps for wild-type (W+) and the 264 A→T mutant are shown.
Figure 7, panels A-C, depict as the upper of the pair of sequences the consensus, wild-type DNA sequence of the *M*. *tuberculosis katG* gene (SEQ ID NO:20), and as the lower of the pair of sequences the amino acid consensus sequence encoded thereby (SEQ ID NO:21). This information is updated from that presented in Figures 1 (SEQ ID NO: 1) and 2 (SEQ ID NO:7) and the numbering system is as used therein. The amino acid and nucleotide sequences are arranged in this figure so as to facilitate convenient determination of which codons encode which amino acid in the polypeptide sequence.
Figure 8 depicts the RFLP patterns produced by an *Msp*I restriction digest of an amplified portion of the DNA of the *katG* genes of wild-type and mutant strains of *M. tuberculosis,* wherein the mutant DNA contains mutations at either codon 315 or codon 463, or both.
Figure 9 depicts the RFLP patterns produced by an *Rsa*I restriction digest of an amplified portion of the DNA of the *katG* genes of wild-type and mutant strains of *M. tuberculosis,* wherein the mutant DNA contain a mutation at codon 337.
Figure 10 depicts the RFLP patterns produced by a *Cfol* restriction digest of an amplified portion of the DNA of the *katG* genes of wild-type and mutant strains of *M. tuberculosis,* wherein the mutant DNA contains a mutation codon 264.

### Detailed Description of the Invention

Wild type strains of *M. tuberculosis* are highly susceptible to isoniazid (INH) with minimum inhibitory INH concentration (MIC or ICₘᵢₙ) ≤ 0.02 µg/ml, and a susceptible strain is considered to be one with an ICₘᵢₙ < 1.0 µg/ml. At the Mayo Clinic, Rochester, Minnesota, clinical strains of *M. tuberculosis* (including MDR-TB strains) were identified which exhibit intermediate to high level resistance to INH (ICₘᵢₙ range 1.0 to > 32 µg/ml). Many of these strains, especially those highly resistant to INH (≥4.0 µg/ml), exhibited diminished catalase activity as assessed by a semiquantitative technique. The mean semiquantitative catalase was 16.5 mm for 6/15 strains with INH ICₘᵢₙ < 1.0 µg/ml and 13.3 mm for 9/15 strains with ICₘᵢₙ ≥ 1.0 µg/ml.

To develop the present assay, it was first necessary to determine whether some M. *tuberculosis* strains have decreased INH sensitivity as a result of *katG* gene mutations. Therefore, the nucleic acid sequences of the *katG* genes for both INH sensitive (ICₘᵢₙ < 1.0 µg/ml) and INH resistant (ICₘᵢₙ ≥ 1.0 µg/ml) *M. tuberculosis* strains were determined. From the DNA sequencing data generated, a *katG* consensus sequence was derived, and *katG* sequences from all 15 *M. tuberculosis* strains (INH sensitive and INH resistant) were compared to the consensus sequence to determine *katG* deviations.

Five of nine INH resistant strains (INH ICₘᵢₙ ≥ 1.0 µg/ml) had one or more missense mutations; one had a nonsense mutation; one had an 8 base pair deletion; and two had no mutations in the coding sequences. All of the five strains with missense mutations had a common G to T transversion at base 1457 in codon 463 (bases 1456-1458) causing replacement of arginine with leucine and loss of an *Nci*I-*Msp*I restriction site. Two of those having mutations at codon 463 also showed a G to C transversion at base 1013 in codon 315 (bases 1012-1014) causing replacement of serine with threonine. A third contained a G to A transversion at base 859 in codon 264 (bases 859-861) resulting in the replacement of alanine by threonine, and a fourth contained an A to G transversion at base 1079 in codon 337 (bases 1078-1080), causing tyrosine to be replaced by cysteine. The numbering system is shown in Figure 1 (SEQ ID NO: 1). The affected codons and portions of the DNA sequences on either side are shown for both INH sensitive and INH resistant strains in Table 2.

Six INH sensitive strains (INH ICₘᵢₙ < 1.0 µg/ml) were also sequenced and found to have from none to 5 amino acid differences with the consensus sequence of all 15 strains, but none of the mutations affected codons 463, 315, 264, or 337 or their overlapping restriction sites. Restriction analysis of a total of 32 sensitive and 43 resistant strains revealed a common restriction fragment length polymorphism (RFLP) in nearly half (19) of the 43 of INH resistant strains, but only one of the INH sensitive strains. Specifically, 44% of the INH resistant had lost the *Nci*I-*Msp*I restriction site at the locus of codon 463 while only 1 of 32 sensitive strains had this restriction polymorphism.

Subsequently, the frequency of codon 463 (R→L) and codon 315 (S→T) mutations in 97 *M. tuberculosis* clinical isolates was determined. These isolates were obtained from patients treated at Mayo Clinic and samples referred from other health care institutions. Restriction fragment length polymorphism (RFLP) analysis using the *Msp*I restriction enzyme, which cleaves at a site spanning the consensus codon 463 site and at a site comprising a portion of the mutant codon 315 site on the *katG* gene of *M. tuberculosis,* was performed on amplified DNA from 97 clinical isolates. Comparison of the resulting RFLP patterns and ICₘᵢₙ for isoniazid revealed that of the 90 INH-resistant strains, approximately 10% had both mutations, 20% had the 315 S→T mutation only, and 26% had the 463 R→L mutation only. Thus, 51 of the 90 resistant strains were identified by RFLP as having mutations at codons 463, 315 or both, resulting in a detection of over 50% of the resistant strains by this molecular method in a single experiment. Only one of the seven INH sensitive strains was found to have the 463 R→L mutation, and none of the INH sensitive strains had the 315 S→T mutation. Greater INH resistance (> 4.0 µg, INH/ml) is associated with the 315 S→T mutation, but not if the 463 R→L mutation is also present.

These results indicate that two mutations, arginine→leucine in codon 463 and serine→threonine in codon 315 of the *M. tuberculosis* catalase-peroxidase (*katG*) gene occur in a significant fraction of INH resistant *M*. *tuberculosis* strains (INH ICₘᵢₙ ≥ 1.0 µg/ml). Furthermore, these single base mutations can be determined using a rapid relatively simple method, i.e., PCR amplification, digestion and monitoring for a loss of an *Nci*I and/or an *Mspl* restriction site at codon 463, and the addition of an *Msp*I restriction site at codon 315, by RFLP, as described in detail hereinbelow. Other restriction endonucleases can be used to determine whether or not these single base mutations exist in *a katG* gene of interest, as long as the restriction site cleaved by the restriction endonucleases contains the affected base, such that the endonuclease cleaves the wild-type sequence but not the corresponding mutant sequence, or vice versa. Although in a preferred embodiment of the invention, the number and location of the fragments is determined by gel electrophoresis, the presence or absence in the digest of a fragment comprising the indicated restriction sites can be determined by other methods known to the art, including immunoassays (dot blots and reverse dot blots), DNA probes, microtiter well capture and the like.

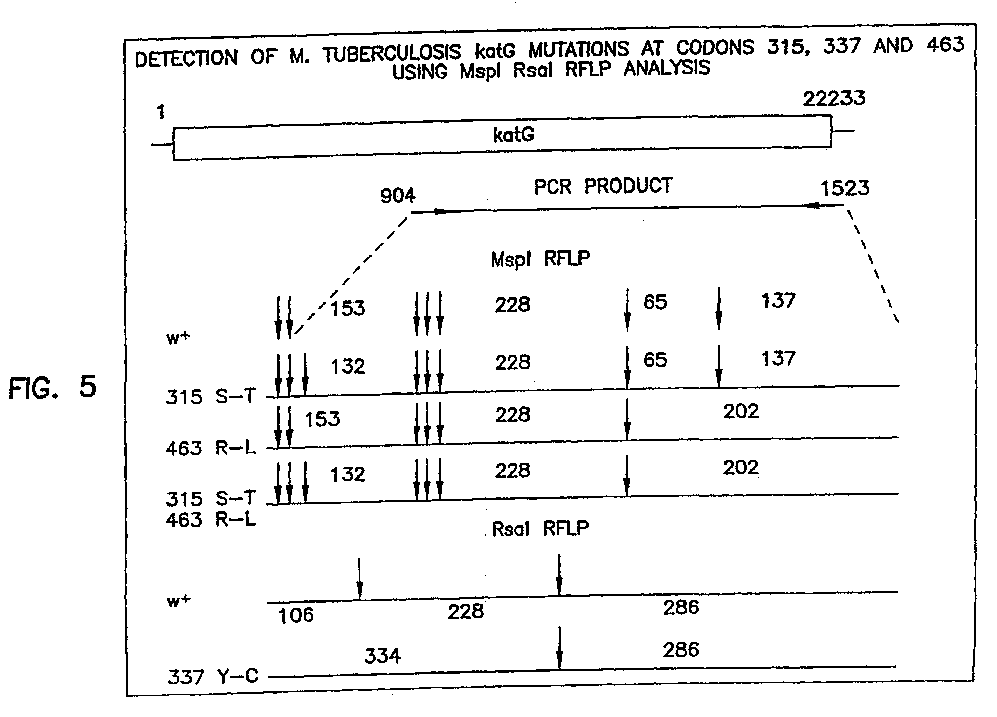

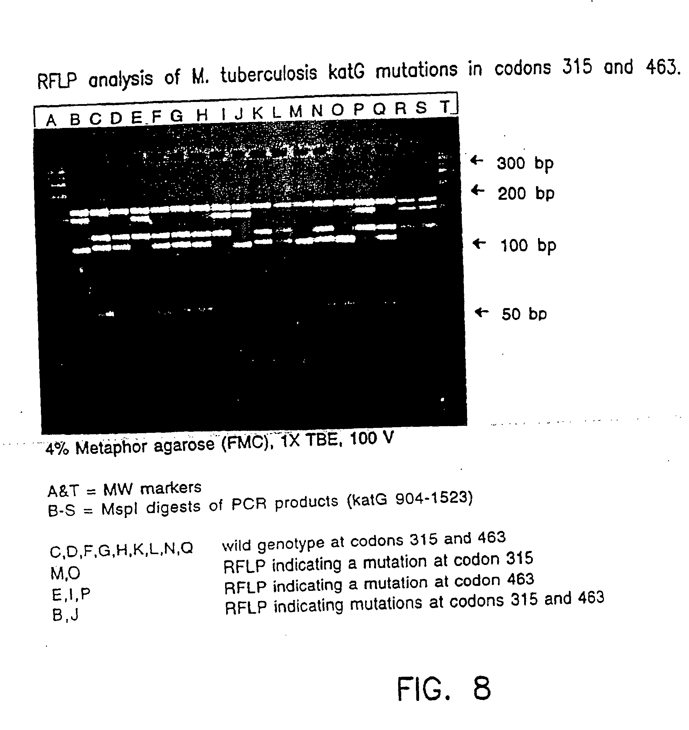

It was further found that the RFLP patterns produced by a *Msp*I digest of the *M. tuberculosis* DNA fragment katG 904-1523 (Figure 8, lanes B-S) are specific for *M. tuberculosis* and thus allow selective detection of *M. tuberculosis. Msp*I digestion of any *M. tuberculosis katG* gene fragment containing the *Msp*I restriction sites depicted in Figure 5 is expected to yield a similar *M. tuberculosis-specific* RFLP pattern. In contrast, *MspI* digestions of DNA samples containing other microorganisms, such as mycobacteria other than *M. tuberculosis* (MOTT), yield either different, distinguishable RFLP patterns, or no detectable restriction fragments at all.

When oligonucleotide primers, preferably katG904 and katG1523, are used in a PCR to generate the 620 base pair *M. tuberculosis* DNA amplicon katG 904-1523 in a sample that contains *M. tuberculosis,* typically the PCR yields no amplicon at all for non-*M. tuberculosis* samples (i.e., the primers do not function to create an amplified product). Thus, the generation of the katG 904-1523 amplicon itself is indicative of the presence of *M. tuberculosis* in the sample. Subsequent enzymatic digestion of the amplicon, preferably using restriction endonuclease *Msp*I, can be used to confirm the determination of *M. tuberculosis*.

The present invention will be further described by reference to the following detailed examples. The 58 clinical strains of *Mycobacterium tuberculosis* used in Examples 1 and 2 were obtained from the Mycobacteriology Laboratory at the Mayo Clinic, Rochester, Minnesota, and the 17 *M. tuberculosis* DNA preparations were obtained from the GWL Hansen's Disease Center, Louisiana State University, Baton Rouge, Louisiana. The strain designated H37Rv MC has been maintained at the Mayo Clinic for over 50 years, and therefore was isolated before INH became available as a treatment modality for tuberculosis (circa 1952). H37Rv was deposited in the American Type Tissue Collection, Rockville, Maryland in 1937 by A. Karlson of the Mayo Clinic under the accession number ATCC 25618, and has been freely available to the scientific community since. An apparent variant of this strain is disclosed in PCT WO 93/22454 (SEQ ID NO: 2, herein). The ATCC strains 27294 and 25618 were recovered from the same patient in 1905 and 1934, respectively. All clinical *M*. *tuberculosis* strains were confirmed as *M*. *tuberculosis* using routine identification techniques described by J.A. Washington, "Mycobacteria and Norcardia," in: *Laboratory Procedures in Clinical Microbiology,* 2d ed., Springer-Verlag, NY (1985) at pages 379-417.

For the *15 M tuberculosis* strains for which complete *katG* DNA sequencing was performed, susceptibility testing was done at the Mayo Clinic using Middlebrook 7H11 agar (DiMed, Inc., St. Paul, Minnesota 55113) and the 1% proportion method described in *Manual of Clinical Microbiology,* 5th ed., A. Balows et al., eds., *Amer. Soc. Microbiol.* (1991) at pages 1138-52. The same method was used at the Mayo Clinic to determine susceptibility for an additional 43 *M. tuberculosis* strains for which restriction fragment length polymorphisms (RFLP) were determined. Isoniazid concentrations tested using this method included: 0.12, 0.25, 1.0, 2, 4, 8, 16, 32 µg/ml for the 15 strains sequenced and 1.0 and 4.0 µg/ml for the remaining 45 strains. Isoniazid resistance was defined as a maximum inhibitory concentration (ICₘᵢₙ) ≥ 1.0 µg/ml. Susceptibility testing was performed elsewhere for an additional 17 *M. tuberculosis* strains for which DNA lysates were provided by Diana L. Williams, Baton Rouge, Louisiana. These strains were of diverse geographical origin. 10 of these 17 strains, originated from Japan. The remaining 7 *M. tuberculosis* INH resistant strains included multiple drug resistant strains from recent multiple drug resistant tuberculosis (MDR-TB) nosocomial epidemics in New York, New York and Newark, New Jersey. All were INH resistant (ICₘᵢₙ ≥ 1.0 µg/ml), and had resistance to at least one other drug. For all strains provided by Williams, the 1% direct proportion method was used, but the concentration of INH tested, and the media used varied as to site.

To conduct a semiquantitative test of catalase activity, *M. tuberculosis* strains were propagated on Lowenstein-Jensen media deeps contained in 20 x 150 mm screw-capped tubes. One ml of a 30% hydrogen peroxide (EM, Science, Gibbstown, New York 08027) and 10% Tween 80 (Aldrich Chemical Co., Milwaukee, Wisconsin 53233) solution mixed in a 1:1 ratio was applied to the surface of growth. After 5 minutes, the highest (mm) of the column of bubbles (O₂) generated was recorded.

### Example 1. DNA Isolation and Polymerase Chain Reaction.

A. DNA Isolation. For *M. tuberculosis* strains obtained from Mayo Clinic samples, DNA was extracted from cells using phenol (Boehringer Mannheim, Indianapolis, Indiana 46250-0414) and TE (1.0 M Tris HCl pH 8.0, 0.1M EDTA, Sigma, St. Louis, Missouri 63778) in a ratio of 600 µl:400 µl and 0.1 mm zirconium beads (Biospec Products, Bartlesville, Oklahoma 74005). The mixture was processed in a mini-bead beater for 30 seconds and allowed to stand for an additional 15 minutes. Following a brief centrifugation to sediment the zirconium beads, DNA in the supernatant was extracted using the IsoQuick kit (MicroProbe Corp., Garden Grove, California 92641).
B. PCR Using Primer Pairs A1-A4 and B1-B2. The DNA sequence for *kat*G (EMBL no. X6808124) employed to design primers is depicted in Figure 1(A-D), lower strand. The PCR method of R.K. Saiki et al., *Science,* 239, 487 (1988) was used to amplify the *katG* gene (ca. 2220 base pairs) in two segments which were designated A and B. Genomic DNA preparations (2 µl) were used with primers A1 (5' TCGGACCATAACGGCTTCCTGTTGGACGAG 3') (SEQ ID NO:3) and A4 (5' AATCTGCTTCGCCGACGAGGTCGTGCTGAC 3') (SEQ ID NO:4) or B 1 (5' CACCCCGACGAAATGGGACAACAGTTTCCT 3') (SEQ ID NO:5) and B2 (5' GGGTCTGACAAATCGCGCCGGGCAAACACC 3') (SEQ ID NO:6).

The PCR mixture (50 µl) contained 10 mM TRIS, pH 8.3, 50 mM KCI, 1.5 mM MgCl₂, 0.2 mM each of dATP, dTTP, dGTP, dCTP, 1 µM of each primer pair, 10% glycerol, 1.25 units/50 µl AmpliTaq DNA polymerase (Perkin Elmer Cetus). The mixture was overlaid with mineral oil and subjected to 4 min at 95°C followed by 50 cycles of 1 min at 94°C and 2 min at 74°C. A 1495 base pair product from the first half of *katG* was generated from the A1-A4 primers and 1435 base pair product was generated with the B1-B2 primer pair.

### Example 2. DNA Sequencing and Homology Analysis.

The polymerase chain reaction (PCR) products were prepared for sequencing using the Magic™ PCR Preps DNA Purification System (Promega Corp., Madison, Wisconsin 53711). The DNA sequences were determined in both directions using the Taq dye-deoxy terminator cycle sequencing kit and 373A DNA sequencer (Applied Biosystems, Foster City, California 94404) using a series of internal sequencing primers which provided appropriate coverage of *katG*.

The sequence data were analyzed using version 7 of the Genetics Computer Group sequence analysis software, as disclosed by J. Devereux et al., *Nucl. Acids Res*., 12, 387 (1984). From the 15 *M. tuberculosis* DNA sequences, a consensus sequence was derived to which all *M. tuberculosis* strains were compared. This consensus sequence is depicted in Figure 1 (A-D) (SEQ ID NO:1) as the upper strand, and is compared to the sequence for *katG* (EMBL no X6808124), depicted as the lower strand. The two sequences have 98.6% identity, as determined by the GCG program BESTFIT. The DNA sequence data has been submitted to Gen Bank and can be referenced by the accession numbers UO6262 (H37Rv MC), UO6258 (ATCC 25618), UO6259 (ATCC 27294), UO6260 (G6108), UO6261 (H35827), UO6270 (L6627-92), UO6271 (L68372), UO6264 (L1 1150), UO6268 (L24204), UO6269 (L33308), UO6265 (L16980), UO6266 (L1781), UO6272 (TMC306), UO6263 (L10373), and UO6267 (L23261). An updated, more complete and accurate *M. tuberculosis katG* gene sequence is presented in Figure 7 (A-C) (SEQ ID NO:20).

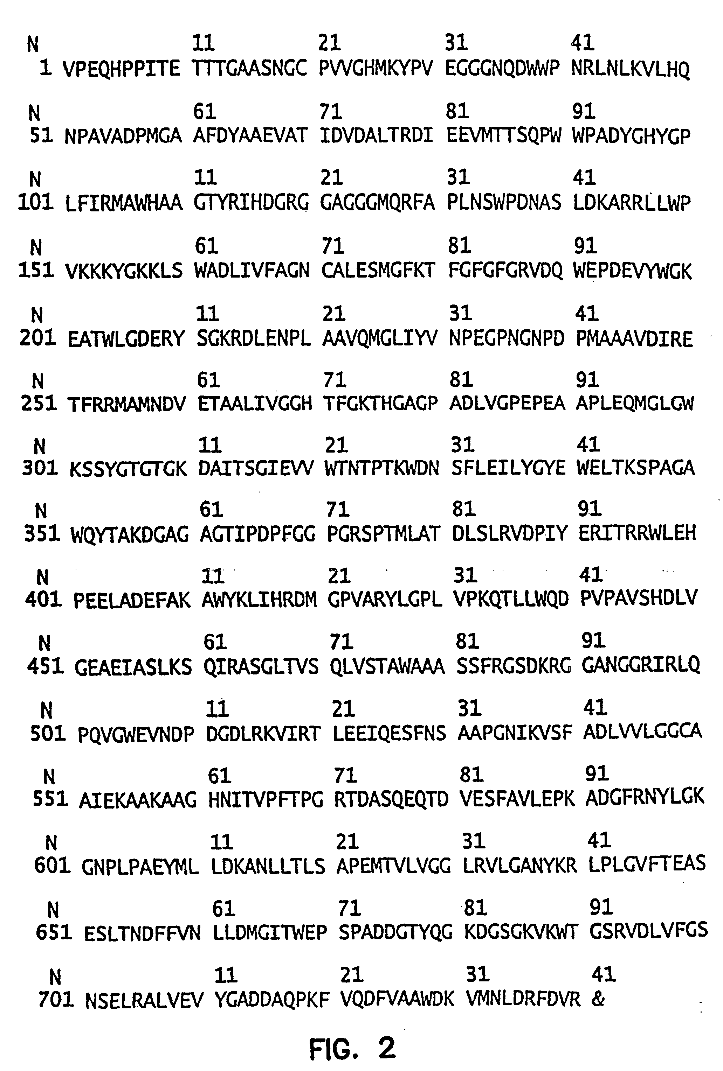

The DNA data was then translated, aligned for comparison and a consensus amino acid sequence was generated (Figure 2) (SEQ ID NO: 7). The consensus amino acid sequence (SEQ ID NO:21) generated from the DNA of SEQ ID NO:20 is also presented in Figure 7.

In general, the overall sequence agreement between INH sensitive and resistant strains was very high; the only deviations are those shown in Table 3.

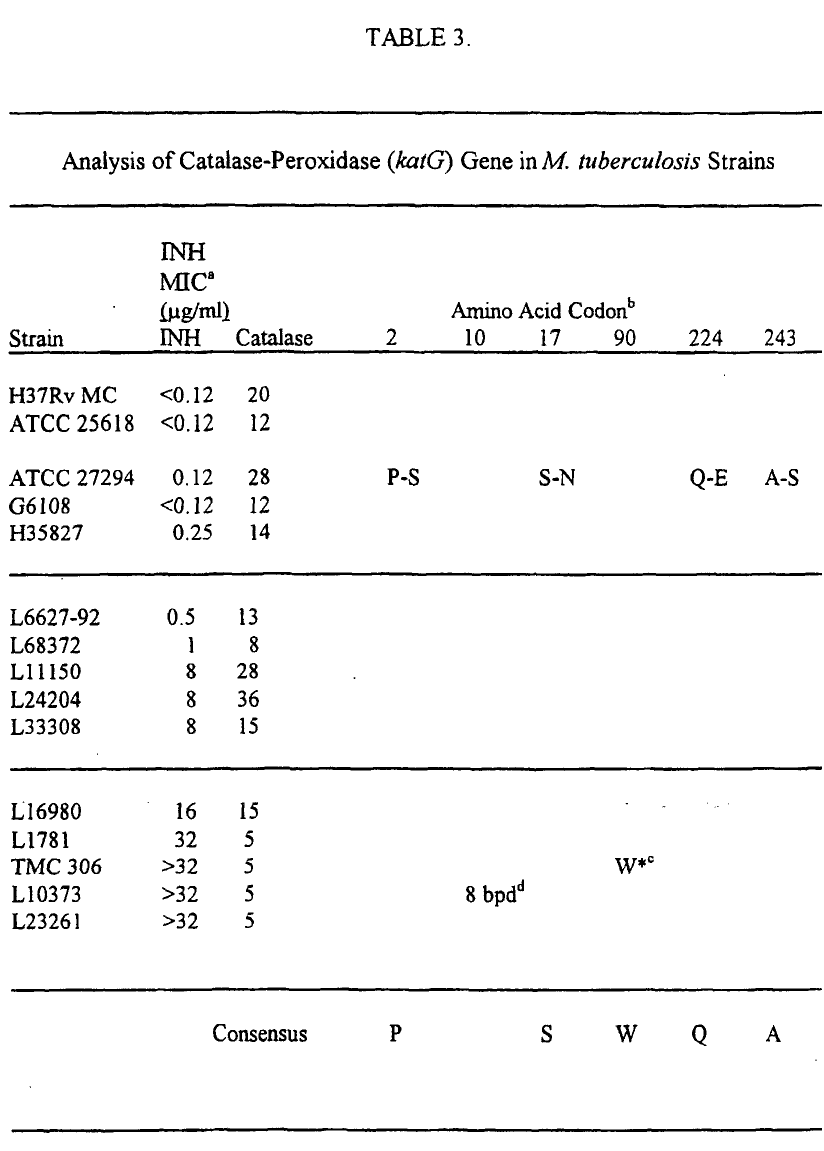

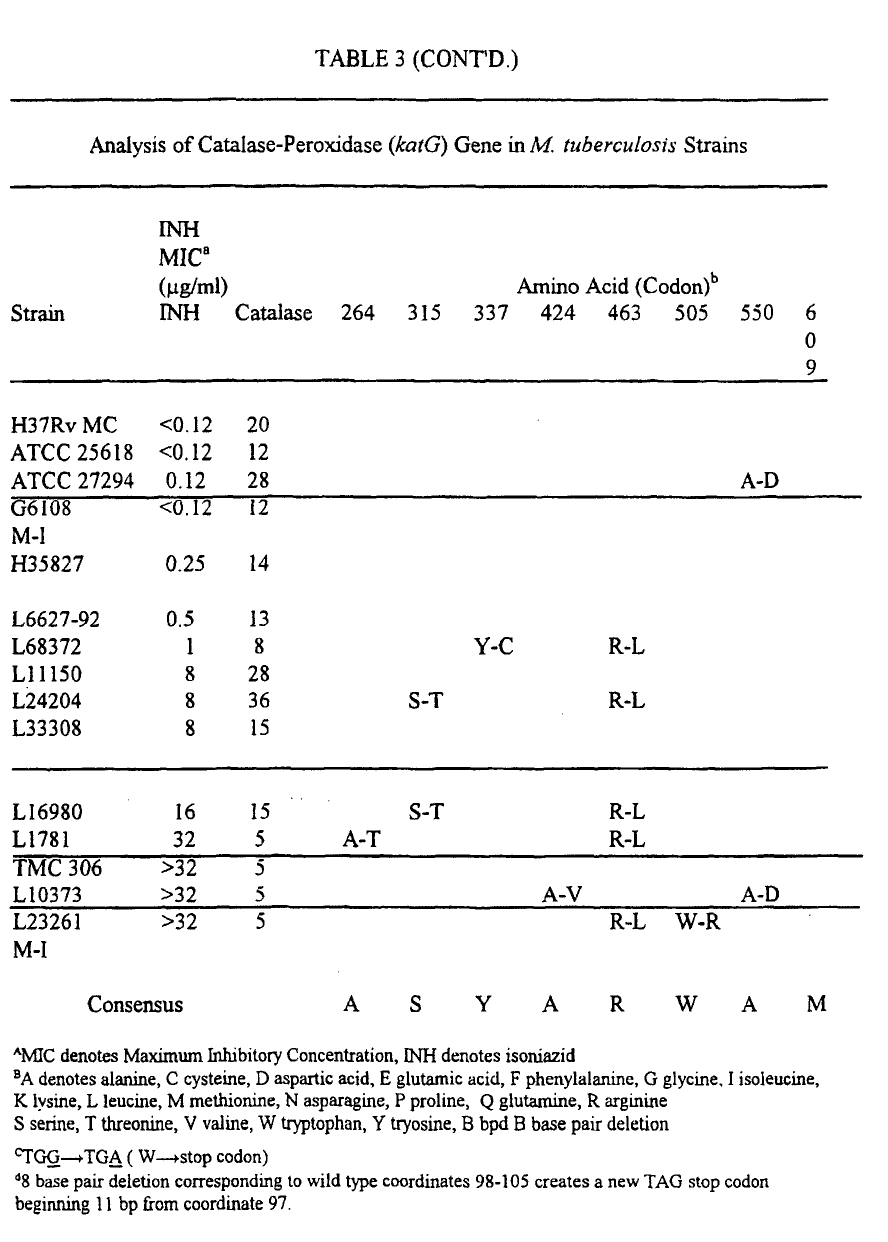

The data in Table 3 show that six strains, H37Rv MC, ATCC 25618, H35827, L6627-92, L11150, and L33308, are completely homologous to the consensus at the indicated sites. Four are INH sensitive (INH ICₘᵢₙ < 1.0 µg/ml) and two are INH resistant (ICₘᵢₙ ≥ 1.0 µg/ml). All other strains listed in Table 3 had 1 to 5 differences with the consensus and there was no strong correlation between the number of differences and INH sensitivity.

In the group of INH resistant strains, the most frequent change observed was the conversion of arginine at codon 463 to leucine. This was detected in five of nine isolates examined. There was not a consistent correlation between the loss of catalase activity and INH resistance since strains L11150 and L24204 had high levels of enzymatic activity, yet were INH resistant. Moreover, several other INH resistant strains showed catalase activity near the mean activity (16.5 mm) of the sensitive strains. Two other isolates had lost the ability to make normal *katG* gene product due either to an eight bp deletion (L10373, semiquantitative catalase, 3mm) or a nonsense mutation (TMC 306, semiquantitative catalase 5 mm). It was not possible to determine if, or how, any of the deviations from the consensus reported in Table 3 affect catalase activity or cause INH resistance. However, the change at codon 463 is frequent enough that is indicative of resistance.

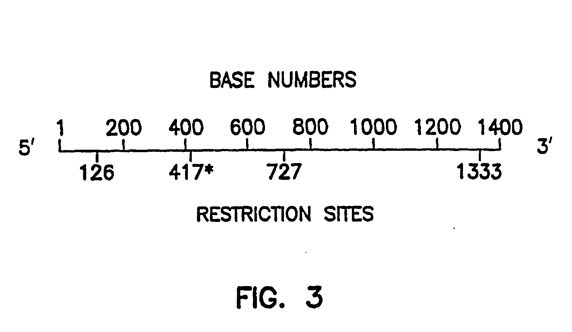

The DNA sequence analysis indicated that the codon 463 occurs in the context of an *Nci*I-*Msp*I restriction site (both enzymes recognize the same site). Thus, when in the wild type sequence depicted in Fig. 1 at bases 1455-1458, CCGGG, is changed to CCTGG, it is no longer recognized (or cleaved) by either of these enzymes. The 1435 bp amplicon produced from the half of *katG* gene containing codon 463 normally has five *Nci*I-*Msp*I restriction sites whereas the codon altered strains have only four sites, as shown in Figure 3. The loss of the site in question causes a unique restriction fragment length polymorphism (RFLP), which can be readily adapted to assay for resistant strains, as described in Example 3, below.

### Example 3. RFLP Analysis: MspI - NciI site in Codon 463

For restriction fragment length polymorphism (RFLP) analysis, a 1435 base pair amplimer (produced using the B1-B2 primers) representing the 3' half of the *katG* gene was generated using PCR and then digested with *Nci*I or *Msp*I (Sigma Chemical Co., St. Louis, Missouri 63178). The gene fragments were analyzed with agarose gel electrophoresis using 2% Metaphor agarose (FMC BioProducts, Richland, Maine 04811). The gel was stained with ethidium bromide and photographed. The investigator who performed all restriction digests and electrophoresis was blinded as to the INH ICₘᵢₙ results.

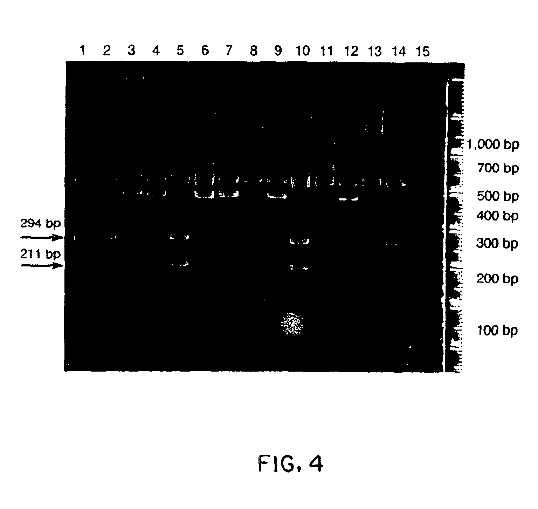

The results of this experiment are depicted in Figure 4, wherein Lane 1 denotes strain H37Rv MC, ICₘᵢₙ = < 0.12 µg/ml; (2) L6627-92, 0.5 µg/mL; (3) L68372, 1.0 µg/ml; (4) L16980, 16 µg/mL; (5) L39791, 16 µg/mL; (6) L1781, 32 µg/mL; (7) L9118, 4 µg/mL; (8) L11150, 8 µg/mL; (9) L24204, 8 µg/mL; (10) L68858, < 0.12 µg/mL; (11) 1115A < 0.12 µg/mL; (12) L23261, > 32 µg/mL; (13) 1341, > 32 µg/mL; (14) M10838, > 32 µg/mL; (15) molecular weight standard: PCR markers (United States Biochemical Corp., Cleveland, Ohio 44122). The digests obtained from resistant strains can be readily visually detected and differentiated from digests from susceptible strains.

Subsequently, a total of 75 *M. tuberculosis* strains (including the 15 strains sequenced) were analyzed for their loss of the appropriate restriction site. Of these strains, 32 were INH sensitive and 43 were INH resistant. The data showed that 19 (44%) of the 43 resistant strains had lost the expected restriction site in codon 463. One of the 33 (2.9%) sensitive strains had lost this restriction sites as well. None of the six sensitive strains listed in Table 3 lost this site.

### Example 4. Determination of the Presence or Absence of Mutations at Codons 264, 315, 337 or 463 in the M. tuberculosis katG Gene

### A. Materials.

Primer pairs used for polymerase chain reaction were katG904/katG1523 (nucleotide sequences 5' AGC TCG TAT GGC ACC GGA AC 3' (SEQ ID NO:16) and 5' TTG ACC TCC CAC CCG ACT TG 3' (SEQ ID NO:17)) and katG633/katG983 (nucleotide sequences 5' CGG TAA GCG GGA TCT GGA GA 3' (SEQ ID NO:18) and 5' CAT TTC GTC GGG GTG TTC GT 3' (SEQ ID NO:19)). Subunits thereof that hybridize to the amplified DNA under the conditions described hereinbelow may also be used.

Polyacrylamide was obtained from National Diagnostics, Tris Borate EDTA solution (6X, cat. no. T6400), magnesium chloride, and dithiothreitol (DTT) from Sigma Chemical Company (St. Louis, MO), TEMED (cat. no. 161-0800) and ethidium bromide (EtBr) from Biorad, ammonium persulfate from Intermountain Sci., and nucleotides (dATP, dGTP, dCTP and dTTP, 100mM solutions) from Boehringer Mannheim Biochemicals. dUTP was obtained from Pharmacia. AmpErase™ uracil-N-glycosylase (UNG) and AmpliTaq™ were obtained from Perkin Elmer.

Restriction endonucleases were obtained as follows: *Msp*I from Sigma (cat. no. R-4506) 10 u/µl with blue palette buffer; *Rsa*I from New England Biochemical (cat. no. 167S) 10 u/µl with NEB buffer 1; and *Cfo*I from Promega (cat. no. R624) 10 u/µl with buffer B.

100 mM nucleotide concentrates obtained from Boehringer Mannheim Biochemicals were used to make the dNTP stock solution, which was 1.25 mM in each nucleotide. Specifically, 10 µl each of dATP, dGTP, dCTP, and dTTP concentrates were added to 760 µl water. dNTP(U) stock solution, also 1.25 mM in each nucleotide, was made from the same 100 mM dATP, dGTP and dCTP concentrates, and 100 mM dUTP concentrate from Pharmacia. Ten µl of each of the four concentrates was added to 760 µl water to make the stock solution.

10X PCR buffer consisted of 100 mM Tris, pH 8.3, 500 mM KCl, and 15 mM MgCl2. PCR mix "A" consisted of 1X PCR buffer, 200 µM each dATP, dGTP, dCTP, and dUTP, 1 µM each katG904 (SEQ ID NO: 16) and katG1523 (SEQ ID NO: 17) primers, 10% glycerol, 10 units/ml AmpErase™UNG, and 0.025 units/µl AmpliTaq. PCR mix B consisted of 1X PCR buffer, 200 µM each dATP, dGTP, dCTP, and dTTP, 1 µM each katG904 (SEQ ID NO: 16) and katG1523 (SEQ ID NO: 17) primers, 10% glycerol, and 0.025 units/µl AmpliTaq. PCR mix "C" consisted of 1X PCR buffer, 200 µM each dATP, dGTP, dCTP, and dUTP, 1 µM each katG633 (SEQ ID NO:18) and katG983 (SEQ ID NO:19) primers, 10% glycerol, 10 units/ml AmpErase™UNG, and 0.025 units/µl AmpliTaq.

Gel loading solution (Blue Juice) was obtained from Sigma (cat. no. G-2526). Gels were photographed on a UV transilluminator (UVP) with Polaroid 667 black and white film (31/4 X 41/4 inch) through an orange filter.

DNA extracts (target DNA) were prepared as described in Example I(A).

### B. MspI RFLP analysis.

PCR was performed by adding 2 µl of DNA extract to 48 µl PCR mix "A." Each reaction was covered with 2 drops of mineral oil. Temperature was cycled (Perkin Elmer DNA Thermo Cycler model 480) for 1 cycle of (5'-37°; 5'-95°) and 40 cycles of ( 1'-94°; 0.5'-60°; 0.75'-72°) and a 72° soak. *Msp*I (10 u/µl) was diluted 1:10 in 100 mM MgCl₂. The amplified DNA (base pairs 904 through 1523) of a wild-type *katG* gene contains 7 *Msp*I restriction sites (Figure 5); of the 8 fragments produced in an *Msp*I restriction digest, 4 are of sufficient length to be visualized using gel electrophoresis (see Figure 5 for a restriction map). Diluted *Msp*I (1 µl) was mixed with 9 µl of the PCR reaction mixture containing the amplified DNA. The digest was incubated at 37°C for 2 hours, then heated to 65°C for 10 minutes. Subsequently, 10 µl of the digest plus 4 µl blue juice was electrophoresed on 6% polyacrylamide for 0.4 hour at 200 V. The gel was stained in EtBr (0.5 mg/ml 1XTBE) for 5 minutes and photographed.

Results are shown in Figure 8. Lanes C, D, F, G, H, K, L, N, and Q show the wild-type genotype at codons 315 (AGC) and 463 (CGG) evidenced by 4 restriction of sufficient length to be visualized using gel electrophoresis (228, 153, 137, and 65 base pairs, respectively, see Figure 5). Lanes M and O show an RFLP indicating a mutation at codon 315 that adds a new *Msp*I restriction site, causing the 153 base pair fragment to be shortened to 132 base pair and become difficult to resolve from the 137 base pair fragment. The resulting 3 fragment pattern (65, 132/137 and 228 base pair) is indicative of an INH resistant strain. Lanes E, I and P show an RFLP indicating a mutation at codon 463 that eliminates an *Msp*I restriction site, evidenced by the 3 visible fragments produced by cleavage versus the 4 produced by the wild-type genotype. The resulting 3 fragment pattern (153, 202, and 228 base pair) is indicative of an INH resistant strain. Lanes B and J show an RFLP indicating mutations at both codon 315 and codon 463. The resulting gain and loss of *Msp*I restriction sites produces a distinctive 3 fragment RFLP pattern (132, 202 and 228 base pair) indicative of an INH resistant strain (see Figure 5 for a restriction map).

### C. RsaI RFLP analysis.

PCR was performed by adding 2 µl of DNA extract to 48 µl PCR mix "B." Each reaction was covered with 2 drops of mineral oil. Temperature was cycled (Perkin Elmer DNA Thermo Cycler model 480) for 1 cycle of 2 minutes at 94° and 40 cycles of (1'-94°; 0.5'-60°; 0.75'-72°) and a 4° soak. *Rsa*I (10 u/µl) was diluted 1:20 in 100 mM MgCl₂/100 mM dithiothreitol. The amplified DNA (bases 904 through 1523) of a wild-type *katG* gene contains 2 *Rsa*I restriction sites. Diluted *Rsa*I (2 µl) was placed on top of the PCR reaction mixture (on the oil) and centrifuged at about 12,000 x g for 10 seconds to drop the *Rsa*I enzyme into the mixture containing the amplified DNA. The resulting mixture was incubated overnight (15-20 hours) at 37°, after which 10 µl of the digest plus 1 µl blue juice was electrophoresed on 6% polyacrylamide for 0.4 hour at 200 V. The gel was stained in EtBr (0.5 mg/ml 1XTBE) for 5 minutes and photographed.

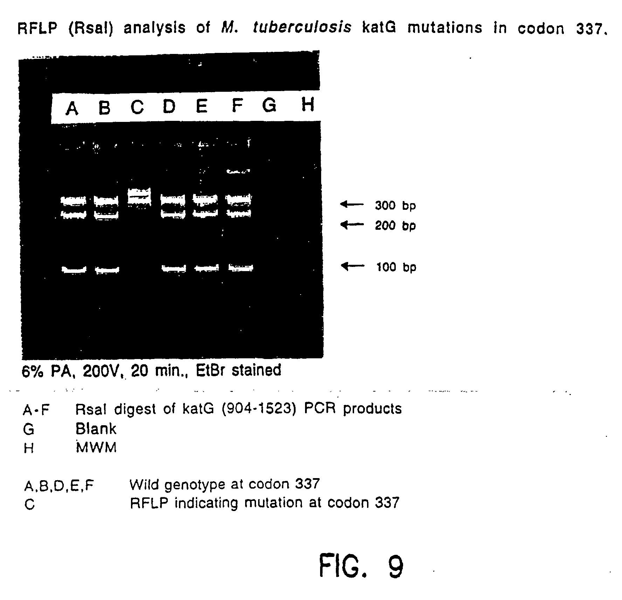

Results are shown in Figure 9. Lanes A, B, D, E, and F show the wild-type genotype at codon 337 (TAC), evidenced by three restriction fragments produced by cleavage at two sites. Lane C shows an RFLP indicating a mutation at codon 337 that eliminates one of the *Rsa*I restriction sites. The resulting two fragment pattern has been observed in an INH resistant strain.

### D. CfoI RFLP analysis.

PCR was performed by adding 2 µl of DNA extract to 48 µl PCR mix "C." Each reaction was covered with 2 drops of mineral oil. Temperature was cycled (Perkin Elmer DNA Thermo Cycler model 480) for 1 cycle of (5'-37°; 5'-95°) and 40 cycles of (1'-94°; 0.5'-60°; 0.75'-72°) and a 72° soak. The amplified DNA (bases 633 through 983) of a wild-type *katG* gene contains 3 *Cfo*I restriction sites. *Cfo*I (10 u/µl) was diluted 1:5 in 100 mM MgCl₂. Diluted *Cfo*I (1 µl) was mixed with 9 µl of the PCR reaction mixture containing the amplified DNA. The digest was incubated at 37° for 2 hours, then heated to 65°C for 10 minutes. Subsequently, 10 µl of the digest plus 4 µl blue juice was electrophoresed on 6% polyacrylamide for 0.4 hour at 200 V. The gel was stained in EtBr (0.5 mg/ml 1XTBE) for 5 minutes and photographed.

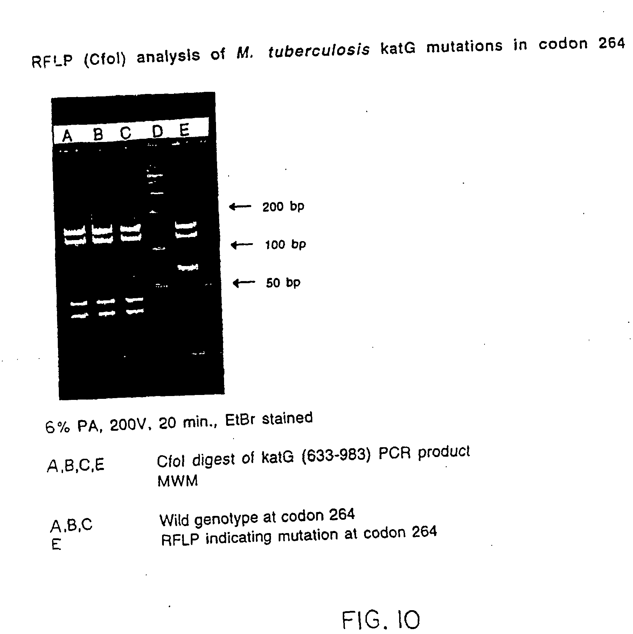

Results are shown in Figure 10. Lanes A-C show the wild-type genotype at codon 264 (GCG), evidenced by 4 restriction fragments produced by cleavage at three sites. Lane E shows an RFLP indicating a mutation at codon 264 that eliminates one of the *Cfo*I restriction sites. The resulting three fragment pattern has been observed in an INH resistant strain.

### Example 5. Rapid Simultaneous Detection of M. tuberculosis (MTB) and Determination of Isoniazid (INH) Susceptibility Directly from Sputum

Five microliter aliquots of 785 ethanol-fixed sputum samples from 365 patients were screened for MTB and INH resistance using the *Mspl* RFLP analysis disclosed in Example 4(B). Primers katG904 (SEQ ID NO: 16) and katG1523 (SEQ ID NO:17) were used in a polymerase chain reaction as described in Example 4 to produce a 620 base pair katG gene fragment or "amplicon" (base pairs 904 through 1523), which was digested with *Msp*I. The resulting restriction fragment pattern was visualized using gel electrophoresis. A result considered "positive for MTB" was defined as production of a katG amplicon that generated an RFLP pattern (see Figure 8) indicative of wild-type MTB or mutant MTB (MTB containing a S315T or R463L mutation, or both, in the 620 base pair katG amplicon). A negative result was defined as the failure to produce a katG amplicon (i.e., failure to produce the 620 base pair segment in the PCR), or, in rare cases, the production of a katG amplicon followed by generation of an RFLP pattern that differed from the RFLP pattern know to be associated with wild-type or mutant (S315T, R463L or S315T/R463L) MTB.

The results of this PCR-RFLP assay were compared to results for acid-fast bacilli (AFB) staining by the Ziehl-Neelsen method, and to results of culture and INH susceptibility testing using the BACTEC radiometric method. Technologists performed PCR-RFLP after AFB bacilli stains and cultures were done, and were blinded to the results for AFB stains and cultures. Patient charts were also reviewed for clinical correlation.

Seventy of 785 (8.9%) sputa were AFB stain-positive. MTB was cultured from 48 of these 70 samples. For 9 other AFB stain-positive samples, MTB was not isolated, however MTB was isolated from these same patients from a recent prior sputum. Eight of these 9 patients were receiving antituberculous therapy at the time the sputum was collected for the study.

Mycobacteria other than MTB (MOTT) were exclusively cultured from 9 other AFB stain-positive specimens [*M*. *avium intracellulare (6), M*. *fortuitum (2), M. kansasii* (1)]. No mycobacteria (MTB or MOTT) were cultured from 2 AFB stain-positive sputa obtained from two patients whose recent prior sputa did not grow mycobacteria. No clinical laboratory information (including whether prior cultures for mycobacteria were done) was available for the 2 remaining AFB stain-positive but culture-negative sputa.

The results for the PCR-RFLP were positive for MTB for 45 of the 48 AFB stain-positive samples that grew MTB. Two of these 3 discordant samples had few AFB on stain and 1 had rare AFB on stain. Additional 5 microliter aliquots from these 3 discordant samples were tested and produced positive results for PCR-RFLP. For the 9 samples that were AFB stain-positive, MTB culture-negative, and where recent prior samples from the same patient were MTB culture-positive, PCR-RFLP results were positive for MTB. In no case was a *katG* amplicon generated for the 9 samples from which MOTT were recovered on culture. PCR-RFLP results were also negative for MTB for the 4 remaining AFB stain-positive, culture-negative samples.

For AFB stain-positive samples from which MTB was isolated and for which susceptibility testing was performed (n=45), 7 isolates (15.6%) were INH resistant. All of these INH resistant isolates were detected by the PCR-RFLP and analysis of the RFLP pattern showed them to carry the S315T mutation. These 7 isolates were from 7 different patients and 4 had different susceptibility patterns for other drugs.

The PCR-RFLP method produced a *katG* amplicon for 39 of the 715 AFB stain-negative samples. MTB was cultured from only 3 of these samples. However, for 21 of the 39 samples, although MTB was not cultured, MTB was recovered from recent prior samples from the same patient and/or the patient was receiving antituberculous therapy at the time the study sample was collected. Comprehensive clinical and laboratory chart reviews were not available for the remaining 15 patients.

This PCR-RFLP MTB *katG* assay, which can be performed in one working day, is thus a reliable, rapid method for detecting MTB and determining INH susceptibility directly from AFB stain-positive sputa.

The invention has been described with reference to various specific and preferred embodiments and techniques.

## Claims

1. A method for selectively identifying *M. tuberculosis* in a sample containing DNA comprising:
amplifying the DNA to generate amplified DNA comprising a detectable amount of *katG* DNA fragment consisting of base 904 through 1523 of the *M. tuberculosis katG* gene as depicted in Figure 7 (SEQ ID NO: 20); and
detecting the presence of the *katG* DNA fragment; wherein the generation of the *katG* DNA fragment is selective for the presence of *M. tuberculosis* in the sample.

2. The method of claim 1 wherein the DNA is amplified in a polymerase chain reaction using oligonucleotide primer katG904 (SEQ ID NO:16) and oligonucleotide primer katG1523 (SEQ ID NO:17).

3. The method of claim 1 or 2 further comprising:
determining whether or not the *katG* DNA fragment has a S315T mutation, wherein the presence of a S315T mutation is indicative of an INH-resistant strain of *M. tuberculosis*.

4. The method of claim 3 wherein the *katG* DNA fragment comprises a restriction site comprising either a G or a C at the nucleotide position occupied by base 1013 in codon 315 of the *M. tuberculosis katG* gene as depicted in Figure 7 (SEQ ID NO: 20), and wherein the step of determining whether or not the *katG* DNA fragment has a S315T mutation comprises contacting the *katG* DNA fragment with a restriction endonuclease that cleaves either at said restriction site comprising a G at the nucleotide position occupied by base 1013 of codon 315, or at said restriction site comprising a C at the nucleotide position occupied by base 1013 of codon 315, but not at both of said restriction sites, to yield at least one cleaved fragment, and wherein cleavage at said restriction site is indicative of either the presence or the absence, but not both, of a S315T mutation in the *katG* DNA fragment.

5. The method of claim 4 further comprising:
electrophoresing the at least one cleaved fragment to yield an electrophoretic mobility pattern comprising the at least one cleaved fragment; and
analyzing the mobility pattern to selectively detect the presence of *M. tuberculosis* in the sample.

6. The method of claim 5 wherein the electrophoresis comprises gel electrophoresis, and wherein the presence of *M. tuberculosis* in the sample is selectively detected using restriction fragment length polymorphism (RFLP) analysis of said electrophoretic mobility pattern.

7. The method of claims 5 or 6 further comprising:
analyzing the mobility pattern to determine the presence or absence of a S315T mutation in the *katG* DNA fragment.

8. The method of any one of claims 4 to 7 wherein the restriction endonuclease cleaves at C/CGG.

9. The method of any one of claims 4 to 8 wherein the restriction endonuclease is MspI.

10. The method of claim 9 wherein the restriction endonuclease is *Msp*I, and wherein cleavage at said restriction site is indicative of the presence of a S315T mutation in the *katG* DNA fragment.

11. The method of any one of claims 1 to 10 wherein the sample is a biological fluid.

12. The method of claim 11 wherein the biological fluid is human sputum.

## Patentansprüche

1. Verfahren zur selektiven Identifizierung von M. tuberculosis in einer DNA-enthaltenden Probe, umfassend:
Amplifizierung der DNA zur Erzeugung amplifizierter DNA, umfassend eine nachweisbare Menge des katG-DNA-Fragments bestehend aus Base 904 bis 1523 des M. tuberculosis-katG-Gens wie in Figur 7 (SEQ ID NO:20) gezeigt; und Nachweis des Vorliegens des katG-DNA-Fragments,
wobei die Erzeugung des katG-DNA-Fragments selektiv für das Vorliegen von M. tuberculosis in der Probe ist.

2. Verfahren nach Anspruch 1, wobei die DNA in einer Polymerase-Kettenreaktion amplifiziert wird unter Verwendung des Oligonucleotidprimers katG904 (SEQ ID NO:16) und des Oligonucleotidprimers katG1523 (SEQ ID NO:17).

3. Verfahren nach Anspruch 1 oder 2, ferner umfassend:
Bestimmen, ob oder ob nicht das katG-DNA-Fragment eine S315T-Mutation aufweist, wobei das Vorliegen einer S315T-Mutation einen INH-resistenten Stamm von M. tuberculosis anzeigt.

4. Verfahren nach Anspruch 3, wobei das katG-DNA-Fragment eine Restriktionsstelle umfasst, die entweder ein G oder ein C an der Nucleotidposition aufweist, die besetzt ist durch die Base 1013 im Codon 315 des M. tuberculosis-katG-Gens, wie in Figur 7 (SEQ ID NO:20) gezeigt, und
wobei der Schritt der Bestimmung, ob oder ob nicht das katG-DNA-Fragment eine S315T-Mutation aufweist, das Inkontaktbringen des katG-DNA-Fragments mit einer Restriktionsendonuclease umfasst, die entweder an der Restriktionsstelle schneidet, die ein G an der Nucleotidposition umfasst, die besetzt ist durch die Base 1013 des Codons 315, oder an der Restriktionsstelle, die ein C an der Nucleotidposition umfasst, die besetzt ist durch die Base 1013 des Codons 315, aber nicht an beiden Restriktionsstellen, um mindestens ein geschnittenes Fragment zu erhalten und wobei das Schneiden an der Restriktionsstelle entweder das Vorliegen oder Fehlen, aber nicht beides, einer S315T-Mutation im katG-DNA-Fragment anzeigt.

5. Verfahren nach Anspruch 4, femer umfassend:
Elektrophoretische Auftrennung des mindestens einen geschnittenen
Fragments, um ein elektrophoretisches Mobilitätsmuster zu erhalten, das das mindestens eine geschnittene Fragment umfasst; und
Analyse des Mobilitätsmusters zum selektiven Nachweis des Vorliegens von M. tuberculosis in der Probe.

6. Verfahren nach Anspruch 5, wobei die Elektrophorese Gelelektrophorese umfasst und wobei das Vorliegen von M. tuberculosis in der Probe selektiv nachgewiesen wird durch Verwendung der Restriktionsfragmentlängen-Polymorphismus (RFLP)-Analyse des elektrophoretischen Mobilitätsmusters.

7. Verfahren nach Anspruch 5 oder 6 ferner umfassend die Analyse des Mobilitätsmusters zur Bestimmung des Vorliegens oder Fehlens von einer S315T-Mutation im katG-DNA-Fragment.

8. Verfahren nach einem der Ansprüche 4 bis 7, wobei die Restriktionsendonuclease bei C/CGG schneidet.

9. Verfahren nach einem der Ansprüche 4 bis 8, wobei die Restriktionsendonuclease MspI ist.

10. Verfahren nach Anspruch 9, wobei die Restriktionsendonuclease MspI ist und
wobei das Schneiden an der Restriktionsstelle das Vorliegen einer S315T-Mutation im katG-DNA-Fragment anzeigt.

11. Verfahren nach einem der Ansprüche 1 bis 10, wobei die Probe eine biologische Flüssigkeit ist.

12. Verfahren nach Anspruch 11, wobei die biologische Flüssigkeit menschliches Sputum ist.

## Revendications

1. Procédé pour l'identification sélective de *M. tuberculosis* dans un échantillon contenant de l'ADN, comprenant :
l'amplification de l'ADN pour engendrer de l'ADN amplifié comprenant une quantité détectable du fragment d'ADN *katG* consistant en les bases 904 à 1523 du gène *katG* de *M. tuberculosis*, comme représenté sur la figure 7 (SEQ ID n° 20) ; et
la détection de la présence du fragment d'ADN *katG*;
dans lequel la production du fragment d'ADN *katG* est sélective pour la présence de *M. tuberculosis* dans l'échantillon.

2. Procédé de la revendication 1, dans lequel l'ADN est amplifié dans une réaction d'amplification en chaîne par polymérase utilisant l'amorce oligonucléotidique katG904 (SEQ ID n° 16) et l'amorce oligonucléotidique katG1523 (SEQ ID n° 17).

3. Procédé de la revendication 1 ou 2, comprenant en outre : le fait de déterminer si le fragment d'ADN *katG* comporte ou non une mutation S315T, la présence d'une mutation S315T étant indicatrice d'une souche INH-résistante de *M. tuberculosis*.

4. Procédé de la revendication 3, dans lequel le fragment d'ADN *katG* comprend un site de restriction comprenant soit une G, soit une C au niveau de la position de nucléotide occupée par la base 1013 dans le codon 315 du gène *katG* de *M. tuberculosis*, comme représenté sur la figure 7 (SEQ ID n° 20), et dans lequel l'étape consistant à déterminer si le fragment d'ADN *katG* comporte ou non une mutation S315T comprend la mise en contact du fragment d'ADN *katG* avec une endonucléase de restriction qui coupe soit au niveau dudit site de restriction comprenant une G à la position de nucléotide occupée par la base 1013 du codon 315, soit au niveau du site de restriction comprenant une C à la position de nucléotide occupée par la base 1013 du codon 315, mais non dans les deux de ces sites de restriction, pour donner au moins un fragment coupé, et dans lequel la coupure au niveau dudit site de restriction est indicatrice de la présence ou de l'absence, mais non des deux, d'une mutation S315T dans le fragment d'ADN *katG*.

5. Procédé de la revendication 4, comprenant en outre :
l'électrophorèse de l'au moins un fragment coupé, pour l'obtention d'un profil de mobilité électrophorétique comprenant l'au moins un fragment coupé ; et
l'analyse du profil de mobilité pour détecter sélectivement la présence de *M. tuberculosis* dans l'échantillon.

6. Procédé de la revendication 5, dans lequel l'électrophorèse comprend l'électrophorèse en gel, et dans lequel la présence de *M. tuberculosis* dans l'échantillon est détectée sélectivement au moyen de l'analyse du polymorphisme de taille de fragments de restriction (RFLP) dudit profil de mobilité électrophorétique.

7. Procédé de la revendication 5 ou 6, comprenant en outre :
l'analyse du profil de mobilité pour déterminer la présence ou l'absence d'une mutation S315T dans le fragment d'ADN *katG*.

8. Procédé de l'une quelconque des revendications 4 à 7, dans lequel l'endonucléase de restriction coupe au niveau de C/CGG.

9. Procédé de l'une quelconque des revendications 4 à 8, dans lequel l'endonucléase de restriction est *Msp*I.

10. Procédé de la revendication 9, dans lequel l'endonucléase de restriction est *Msp*I et dans lequel la coupure au niveau du site de restriction est indicatrice de la présence d'une mutation S315T dans le fragment d'ADN *katG*.

11. Procédé de l'une quelconque des revendications 1 à 10, dans lequel l'échantillon est un liquide biologique.

12. Procédé de la revendication 11, dans lequel le liquide biologique est un crachat humain.
